# EUROPEAN PATENT APPLICATION

(11) **EP 4 595 866 A2**
(43) Date of publication of application: **06.08.2025**
(21) Application number: 25183816.5
(22) Date of filing: 20.12.2021
(51) Int. Cl.: A61B 1/253

(54) **INTRAORAL SCANNER COMPRISING A DEFOGGING SYSTEM**

(30) Priority: 22.12.2020 DK PA202070862
(62) Divisional of application: 21843902.4
(71) Applicant: 3Shape A/S, 1060 Copenhagen K (DK)
(72) Inventor: HANSEN, Kasper Krogh, 1060 Copenhagen K (DK); SUNDBERG, Oliver, 1060 Copenhagen K (DK); CHRISTIANSEN, Alexander Bruun, 1060 Copenhagen K (DK); HERMANNSSON, Pétur Gordon, 1060 Copenhagen K (DK); KIRSANSKE, Gabija, 1060 Copenhagen K (DK)
(74) Representative: Guardian IP Consulting I/S

(57) **Abstract**

Disclosed is an intraoral scanning system that includes a housing and a sleeve. The housing includes optical components, a head, and a defogging unit that includes a heating unit. The head includes a primary aperture and is configured to be inserted into an oral cavity of a patient. The sleeve includes a secondary optical component arranged at a secondary aperture that is configured to be positioned in alignment with the primary aperture when the sleeve is coupled with the housing. The heating unit is configured to generate heat in response to application of electrical power to the heating unit. When the sleeve is coupled with the housing, the secondary optical component is configured to be arranged such that the generated heat is transferred from the heating unit to the secondary optical component through thermal conduction between the heating unit and secondary optical component.

## Description

### Technical field

The disclosure relates to a defogging system. In particular, the disclosure relates to an optical scanning system comprising the defogging system. The optical scanning system includes a 3D scanner for scanning patient's bodily orifices. For example, the 3D scanner may include a handheld 3D intraoral scanner for scanning patient's oral cavity.

### Background

Medical devices such as 3D intraoral scanners that are inserted in a patient's bodily orifice such as an oral cavity generally operate in an environment with high requirements to hygiene and/ or exposed to humid and warm environment. So, condensation is likely to occur on scanning system surfaces such as surfaces that, prior to insertion into the body orifice, were at ambient temperature. For example, 3D intraoral scanners are regularly exposed to exhaled breath of the patient or the air in the oral cavity that causes fogging of the surfaces such as optical components inserted into the oral cavity. Such condensation on the optical components used in imaging the bodily orifice may interfere with the optical operation of the medical device. For example, condensation on the inserted optical components may cause an undesired change such as altering the scanning signal (e.g. in path or transmission of the light signal, i.e. illuminating light or reflected light), resulting in significant errors in acquired data or acquired images with degraded image quality.

Several defogging systems are known to defog the insertable surfaces (e.g. optical components). One conventional defogging system includes an airflow system that supplies cold or warm air to the insertable surfaces. In sensitive patients such as having sensitive tooth, the flow of air may cause discomfort. The generation of an airflow also leads to increased power consumption and noise emissions. In addition, the required air-pump or nozzle needed for this purpose takes up space in the medical device. In another defogging system such as used in an intraoral scanner having two-windows system, an outer window exposed to the bodily environment is defogged by way of heat transfer by radiation across a gap between the outer window and an inner window that gets heated up by a heater. The effectiveness (e.g. defogging time) may depend on certain factors such as design of the windows system, choice of materials, etc. Additionally, the effectiveness of such a defogging system is hugely influenced by a distance between the inner window and outer window, i.e. gap width that is aimed to be kept as small as possible. There are limitations such as manufacturing tolerances determining how small the gap width can be - the greater the gap width is, the lesser effective such defogging system becomes. Even for a small gap width (e.g. 0.5 mm), the defogging time is usually in an order of magnitude that delays making an optical scanning system having these defogging systems ready for scanning a bodily orifice or during the scanning process. Further, these defogging systems may become undesirable for battery operated optical scanning systems, which require an efficient use of limited battery power and a shorter defogging time.

Additionally, outer surfaces of the insertable part (e.g. scanning system surfaces) of the medical device need to create a separation between the patient and internal units or components of the medical device. This prevents contamination of the internal units or components of the medical device, thus allowing the medical device to be used across different patients while still maintaining hygiene. To further comply with hygienic requirements, the outer surfaces of the insertable part (e.g. scanner sleeve or outer window) need to be generally removable and may typically be required to be either disposed and replaced by another insertable part such as another sleeve or may need to be sterilized (e.g. hot air sterlization, steam autoclave, or chemical sterlization) for reuse in another patient.

Thus, it is desirable to provide an optical scanning system (e.g. battery-operated intraoral scanning system) comprising a defogging system, which overcomes at least some of the above shortcomings while satisfying hygienic requirements to allow repeated use of the medical device (e.g. intraoral scanner) across different patients.

### Summary

According to an embodiment, an intraoral scanning system is disclosed. The intraoral scanning system includes a housing and a sleeve. The housing includes optical components configured to acquire data comprising a plurality of two-dimensional images in response to illumination of a dental object. The sleeve is configured to cover at least a part of the housing when the sleeve is coupled with the housing.

The intraoral scanning system disclosed in the above embodiment is configured to be combined with one or more embodiments disclosed later.

According to an embodiment, an intraoral scanning system is disclosed. The intraoral scanning system includes a housing and a sleeve. The housing includes optical components, a head, and a defogging unit that includes a heating unit. The head includes a primary aperture and is configured to be inserted into an oral cavity of a patient. The sleeve includes a secondary optical component arranged at a secondary aperture that is configured to be positioned in alignment with the primary aperture when the sleeve is coupled with the housing. The heating unit is configured to generate heat in response to application of electrical power to the heating unit. When the sleeve is coupled with the housing, the secondary optical component is configured to be arranged such that the generated heat is transferred from the heating unit to the secondary optical component through thermal conduction between the heating unit and secondary optical component.

According to another embodiment, an intraoral scanning system is disclosed. The intraoral scanning system includes a housing and a sleeve. The housing includes optical components, a head, and a defogging unit that includes a heating unit. The head includes a primary aperture and is configured to be inserted into an oral cavity of a patient. The sleeve is coupled with the housing and covers at least a part of the head. The sleeve includes a secondary optical component arranged at a secondary aperture, which is positioned in alignment with the primary aperture. The heating unit is configured to generate heat in response to application of electrical power to the heating unit. The secondary optical component is arranged such that the generated heat is transferred from the heating unit to the secondary optical component through thermal conduction between the heating unit and secondary optical component.

Transferring the generated heat from the heating unit to the secondary optical component through thermal conduction may also include thermal conduction of the transferred heat over and/ or across the secondary optical component. This is achieved, as an example, by having the secondary optical component made up of a material having high thermal conductivity.

When the sleeve is coupled with the housing, the sleeve covers at least a part of the head and the secondary optical component typically gets arranged in relation to the housing. This arranging of the secondary optical component allows the generated heat to get transferred from the heating unit to the secondary optical component through thermal conduction between the heating unit and secondary optical component.

The sleeve is configured to cover at least a part of the head. As the sleeve is configured to cover at least a part of the head when coupled with the housing, the sleeve is therefore also configured to provide at least one of mechanical protection to at least some components enclosed within the housing, or a hygiene barrier between the patient and at least a part of the head. The sleeve may allow keeping the covered part of the head contamination free or at least reduce exposing the covered part of the head to the environment in the oral cavity.

The term aperture may be understood as a space through which the scanning signal passes. For example, this may include a hole, opening or gap.

The optical components include optical elements for imaging the intraoral cavity. These optical elements are generally configured to direct light out of the scanner for scanning such as towards an intraoral cavity of patient for illuminating the intraoral cavity, and to direct, towards a sensor, light entering the scanner after the illuminating light gets reflected from the intraoral cavity. Directing the illuminating light and/ or reflected light may be achieved by a relative arrangement of the light source in the dental scanning system with respect to a scanner head of the scanning system and sensor. As an example, the optical components incudes one or more of light source, prism, mirror, lens system, mirror, beamsplitter, etc. The referred illuminating light and/ or the reflected light may be understood as a scanning signal.

Scanning or scanning procedure includes at least the steps of illuminating the oral cavity and receiving data, by way of the reflected light, at the sensor but may also include one or more steps that utilizes the received data, such as the step of processing the received data, to generate a three-dimensional digital representation of the oral cavity.

The housing includes a volumetric space having at least an illumination unit, sensor, and processor within the space. The illumination unit comprising the light source is configured to illuminate the intraoral cavity (e.g. comprising a three dimensional dental object), the sensor is configured to acquire data comprising a plurality of two-dimensional images in response to the illumination of the dental object; and the processor is configured to generate processed data by processing one or more of acquired data, wherein a three-dimensional digital representation of the dental object is generated based on the processed data. The three-dimensional dental object that is imaged using the scanning system may include an intraoral cavity comprising one or more of patient's teeth, gingival tissue, edentulous areas, or any other material like restoration, implant, etc. The acquired data may include information relating to the three-dimensional dental object such as 2D images, depth data and may additionally include at least one of a color data or a data that is obtainable from the depth data and/ or color data such as surface color date, fluorescence color data or infrared light data. The sensor is an image sensor that is configured to generate the plurality of images based on the incoming (reflected) light received from the illuminated dental object. The processor is configured to generate the processed data by processing one or more of the plurality of two-dimensional images. The processed data may include information relating to depth data representing surface topology of the three-dimensional dental object, and may additionally include information relating to color data representing color of the three-dimensional dental object.

In an embodiment, the intraoral scanning system is configured to transmit, wirelessly or through a wired connection, the processed data to a remote processing unit that may be part of the intraoral scanning system. The remote processing unit is configured to generate the three-dimensional digital representation of the dental object based on the received processed data. Thus, the processing may include making the processed data suitable for transmission such as wireless transmission. This may include at least one of filtering, compressing, encrypting, or encoding the information available in the one or more of the plurality of two-dimensional images, and output of such processing represents the processed data. The handheld intraoral scanning system may include wireless functionality, which may be achieved by an in-built wireless capability within the housing or by way of a connection with a wireless enabled unit. In another embodiment, the processor comprised in the housing is configured to generate the three-dimensional digital representation of the dental object based on the received processed data.

The intraoral scanning system may further include a display, which is configured to display the three-dimensional digital representation of the dental object.

The intraoral scanning system may operate using a scanning principle such as triangulation-based scanning, confocal scanning, focus scanning, Optical Coherence Tomography (OCT) scanning, etc. In an embodiment, the scanning system is operated by translating a focus plane along an optical axis of the scanning system and capturing the plurality of 2D images at different focus plane positions such that each series of captured 2D images corresponding to each focus plane forms a stack of 2D images. In other words, the focus plane position is preferably shifted along the optical axis of the scanning system, such that 2D images captured at a number of focus plane positions along the optical axis forms said stack of 2D images (sub-scan) for a given view of the object, i.e. for a given arrangement of the scanning system relative to the object within a short time interval. After changing the arrangement of the scanning system relative to the object a new stack of 2D images for that view may be captured. The focus plane position may be varied by means of at least one focus element, e.g., a moving focus lens. The 3D scanner is generally moved and angled such that at least some sets of sub-scans overlap at least partially, in order to enable stitching. The result of stitching is the digital 3D representation of a surface larger than that which can be captured by a single sub-scan, i.e. which is larger than the field of view of the 3D scanner. Stitching, also known as registration, works by identifying overlapping regions of 3D surface in various sub-scans and transforming sub-scans to a common coordinate system such that the overlapping regions match, finally yielding the overall scan. The Iterative Closest Point (ICP) algorithm is widely used for this purpose. In another embodiment, the scanning system such as the intraoral scanner may employ conventionally known triangulation-based scanning using a structured probe light defined by a pattern, thereby illuminating the pattern on the object and receiving the reflected signal in response to the illumination as plurality of 2D images at the sensor. The received images are then processed to generate the digital 3D representation of the illuminated object, i.e. of the intraoral cavity.

In an embodiment, the intraoral scanning system comprises a power interface that is configured to receive operating power directly from plug-socket based mains electricity. Additionally or alternatively, the intraoral scanning system comprises a battery slot that receives a removable, usually rechargeable, battery that is configured to supply operating power to the intraoral scanning system. In another embodiment, the intraoral scanning system includes an inbuild integrated battery with a charging interface configured to receive recharging power for the battery such that the integrated battery may be recharged. The disclosed defogging unit comprising the heating unit may be used in any of these intraoral scanning systems irrespective of how the operating electrical power is supplied. The disclosed defogging unit by way of thermal conduction may reduce power consumption for defogging and significantly reduces the defogging time, therefore the disclosed defogging unit is particularly useful for an intraoral scanning system that receives its operating power from a limited power source such as a battery. The need for a more effective defogging unit, which results in a reduced power consumption for defogging time may further be useful for an intraoral scanning system that is configured to perform additional tasks such as wirelessly transmit the processed data.

The head may be fixedly attached, i.e. integrated part of the housing. Alternatively, the head may be removably attached to rest of the housing, i.e. the head is configured to be mounted onto or dismounted from the rest of the housing (e.g. a mounting portion of the housing). The mounting and dismounting at the least refers to engaging and disengaging (such as locking and unlocking) of the head with the mounting portion. The mounting portion includes at least a section of the housing to which the head is mounted or dismounted from. The section may include a locking unit that is configured to engage with a complimentary locking unit of the head to allow locking the head with the section. In some embodiments, the mounting portion may include a tube onto which the head may be mounted onto and un-mounted from usually by way of a sliding motion along a length of the mounting portion. In other words, the mounting portion may include a tube-like structure and the head, structured like a hollow tube, is slidably attached to the tube-like mounting structure. As stated earlier, the mounting portion may at least include a locking unit that is configured to engage with a complimentary locking unit of the head to allow locking the head with the mounting portion. In several embodiments, mounting the head onto the rest of the housing establishes a stable mechanical connection between the head and rest of the body. Typically, the intraoral scanning system is made available to the user of the intraoral scanning system with the head already in the mounting position, but the head may be dismounted for servicing the scanner.

The head is configured to be brought into proximity with the body orifice when acquiring oral cavity data during scanning such as for recording the topographic characteristics. This may require at least one optical element of the head (e.g. primary aperture) being inserted in the bodily orifice immediately prior or during the scanning. In some embodiments, the head may be disinfected by wiping such as with a dry cloth/ cleaning wipes or using a cleaning solvent.

In an embodiment, the head includes at least one optical element, and a framework. The at least one optical element such as a prism, or mirror is configured to be inserted into the oral cavity immediately prior or during the scanning. The prism or mirror is configured to direct the scanning signal out of the scanner and/ or towards the sensor. The framework of the head is made up of at least in part with a non-conductive material such as a plastic material (for example, by injection molding). Several plastic materials exist suitable to be used for the head and even for heads that may require autoclaving, for example PSU. With an appropriate glue, the mirror may be glued to the framework. Another method of fixing the mirror to the framework is to weld a thin sheet made of some other material, e.g., metal to the plastic material and use glue to bond the mirror to the sheet. There is a much wider choice of glues (even autoclavable gules, if needed) that bond metal to glass.

In an embodiment, the head includes i) the framework comprising a first opening configured to engage with the mounting portion of a main body of the housing, and ii) a connection component comprising a second opening (i.e. the primary aperture) at a distance from the first opening and configured to allow the illuminating light to pass through the primary aperture to exit the housing and/ or the reflected light to pass through the aperture to enter the housing. The typical position of the first opening and the second opening is such that illuminating light and/ or reflected light passes through one opening and is reflected by the optical element (e.g. mirror in the head) prior to passing through the another opening.

In an embodiment, the head includes a primary optical component arranged at the primary aperture. The primary optical component arranged at the primary aperture is inhibited from such as prevented from receiving the generated heat from the heating unit to the primary optical component. The term arranging the primary optical component may refer to at least one of physical arrangement, physical structure or material property that inhibits such as prevents transfer of the generated heat to the primary optical component. The primary optical component is permanently arranged or removably arranged at the primary aperture, wherein the arranging includes attaching the primary optical component at the primary aperture. The removably arranged primary optical component may allow for replacing a defective primary optical component.

The term "inhibit" or other variations thereof such as "inhibited" refers to hindering or restraining the heat generated by the heating unit, i.e. generated heat from reaching or transferring from the heating unit to the primary optical component. Despite "arranging the primary optical component" as mentioned in the previous paragraph, some heat may still be received at the primary optical component and the primary optical component is gradually heated up. Similarly, despite the gap width, the primary optical component may receive, from the heated secondary optical component, heat via convection or radiation across a gap width defined between the primary and secondary optical components and the primary optical component may gradually heat up. Such heating up of the primary optical component during the scanning procedure may be beneficial because the heated up optical components may remove any humidity trapped between the gap defined by the primary optical component and secondary optical component, thus avoiding in-gap condensation. Thus, the heated up primary optical component in combination with the heated up secondary optical component ensures that, during the scanning procedure that is typically initiated and performed after initial defogging of the secondary primary component, the scanning signal is prevented from getting altered due to the condensation. The heating up of the optical components during the scanning include thermalizing the optical components to an above dew point temperature. Although the primary optical component has a low thermal conductivity, the primary optical component preferably includes material having high heat capacity. The high heat capacity allows the primary optical component to retain heat for a long time, for example long enough to retain heat if the scanner is temporarily switched off for a short while (e.g. changing battery for a battery operated scanner, or other momentary interruptions), to efficiently handle any humidity which may be present between the gap during the scanning procedure. In one embodiment, the primary optical component may be made up of a material such as a glass (e.g. BK7 glass from Schott AG) that has a higher heat capacity than the heat capacity of the secondary optical component that may be made up of a material such as Sapphire. For example, the specific heat of the BK7 glass 0.858 J/g°C at 20°C, whereas that of the Sapphire stays below 0.8000 J/g°C for a positive temperature below 36°C (e.g. 0.7572 J/g°C at 16.85°C and 0.7788 J/g°C at 26.85 °C).

With the fixed head or removable head in the mounted position over the mounting portion, and primary optical component arranged at the primary aperture; the space contained within the housing is closed and prevented from being physically exposed to the environment such as to the oral cavity. This allows for reducing, or even eliminating a risk of contamination of components within the housing.

In one embodiment, arranging the primary optical component to inhibit such as prevent transfer of the generated heat from the heating unit to the primary optical component includes arranging an insulating material between the heating unit and the primary optical component. The insulating material creates a thermal barrier, thereby reducing or even avoiding the heat transfer. The insulating material may be selected from a glue, a plastic, or any other material. The insulating material that has a lower/ poorer thermal conductivity when compared to that of the i) connection component, or ii) connection component and secondary optical component. The insulating material may be selected from an epoxy, Ultraviolet (UV) glue, adhesive or a composite thereof. The thermal conductivity of the insulating material may typically be lower than 1.5Wm⁻¹K⁻¹ at room temperature.

Additionally or alternatively, the arranging the primary optical component to inhibit such as prevent transfer of the generated heat from the heating unit to the primary optical component may include having the primary optical component that is made up of a solid material having a low/ poor thermal conductivity. Thus, use of such material inhibits such as prevents the generated heat is from heating up the primary optical component, i.e. the generated heat is inhibited from such as prevented from transferring from the heating unit to the primary optical component. The thermal conductivity of the primary optical component material is lower than that of the i) connection component, or ii) connection component and secondary optical component. The primary optical component may be selected from transparent glass, plastic, a composite thereof. The thermal conductivity of the primary optical component material may typically be lower than 1.5Wm⁻¹K⁻¹ at room temperature.

Additionally or alternatively, the arranging the primary optical component to inhibit such as prevent transfer of the generated heat from the heating unit to the primary optical component may include a substrate having a deposit of poor thermal conductivity disposed on the primary optical component such as on at least one of a primary first surface or a primary second surface of the primary optical component or surfaces defining the edge width. Thus, use of such deposit inhibits such as prevents the generated heat from heating up the primary optical component, i.e. the generated heat is inhibited from such as prevented from transferring from the heating unit to the primary optical component. The thermal conductivity of the deposit material is lower than that of the i) connection component, or ii) connection component and secondary optical component. The substrate may be selected from transparent glass, plastic, a composite thereof, and may include the primary optical component itself. The deposit material may have a thermal conductivity that is lower than may typically be lower than 1.5Wm⁻¹K⁻¹ at room temperature.

Additionally or alternatively, the arranging the primary optical component to inhibit such as prevent transfer of the generated heat from the heating unit to the primary optical component may include the primary optical component including a multi-layered structure where at least two layers, which may be a deposit layer, of the multi-layered structure have different thermal conductivity and/ or optical properties. The multi-layered structure includes a plurality of layers that are arranged over one another, i.e. overlaid, along a propagation direction of the scanning signal and/ or a plurality of layers that are arranged adjacent to one another along a surficial dimensions of the primary optical component. In an embodiment, one of the adjacent layers of the primary optical component is made up of an insulating material, which acts as a thermal barrier between the heating unit and other layer(s) of the primary optical component. The other layer(s) may be overlaid layers, adjacent layers of combination thereof. The insulating adjacent layer may be a layer that is first exposed to the generated heat compared to other layers of the primary optical component. The insulating layer thus creates a thermal barrier, thereby reducing or even avoiding the heat transfer. In an embodiment, it may be preferable that all the overlaid layers of the primary optical component are made up of an insulating material to avoid heating of the primary optical component, however it is also possible to have any number of (i.e. one or more) layers of the plurality of layers to be made up of the insulating material. This may be particularly useful if the primary optical component is required to have a specific thermal and/ or optical properties while still inhibiting such as preventing the generated heat getting transferred to the primary optical component.

This insulating adjacent layer may be a layer that is first exposed to the generated heat compared to other layers of the primary optical component. The insulating layer thus creates a thermal barrier, thereby reducing or even avoiding the heat transfer.

In any of the above embodiments, the layers may be a deposit material and/ or a layer with a predefined thickness. The thermal conductivity of the insulating adjacent layer or deposit material may comply with the thermal conductivity conditions in relation to the insulating material or primary optical element or deposit, as disclosed above.

The primary optical component includes optical properties that allow the scanning signal to pass through the primary optical component such that the oral cavity may get illuminated and the sensor may receive the light reflected from the oral cavity in response to the illumination. Typically, the primary optical component may be selected from a transparent glass, plastic, a composite thereof or any other material that allows transmission of the scanning signal without substantial alteration to the scanning signal to avoid errors in acquired data or acquired images. The primary optical component includes the primary first surface or the primary second surface, wherein the primary first surface and the primary second surface are opposite surfaces of the primary optical component with one surface facing the volumetric space of the housing and the other surface facing the secondary optical component when the sleeve is coupled with the housing.

In an embodiment, the housing such as the head of the housing includes a connection component that is configured to establish a physical connection between the heating unit and the secondary optical component to permit transfer of the generated heat by thermal conduction from the heating unit to the secondary optical component. The connection component is typically in a physical connection with the framework of the head. In one embodiment, the at least a part of the connection component is arranged on the surface of the framework. In another embodiment, the connection component is attached at its edges with the framework.

The term "physical connection" or "physically connected" or a variation thereof in relation to the connection component and secondary optical component is used to define thermal connection by way of any of direct physical contact or physical contact via an intermediate component that is typically a solid component unless specifically described otherwise. Similarly, use of the term conductive or insulating or variations thereof refer to thermal conducting or insulating properties unless described otherwise.

In any of the above embodiment, the connection component may include at least one plate such as a pair of plates or a structure including interconnected plates. Irrespective of number of plates, the connection component is dimensioned and/ or shaped to prevent from interfering with the scanning signal, i.e. prevented from obstructing the scanning. The thermal conductivity of the connection component is higher than that of the arranged primary optical component. The arranged primary optical components refer to different embodiments describing "arranging the primary optical component", as disclosed earlier. The connection component is made up of a solid material having a thermal conductivity higher than 7 Wm⁻¹K⁻¹ at room temperature. The connection component is usually made up of metal such as Aluminum but other solid material like metals or alloys thereof having high thermal conductivity may also be used. In an embodiment, the connection component may be provided with an anodization layer such as a black anodization layer that is configured to avoid stray light inside the scanner. The anodization layer, typically a thin layer, may have a low thermal conductivity, but the connection component that is made up of a solid material having a thermal conductivity allows for efficient transfer of the generated heat from the heating unit to the secondary optical component. Thickness of the anodization layer may vary typically in the range of 0.5 µm to 100 µm.

Apart from the heating unit, the defogging unit may include an electrical interface that is configured to receive electrical power in form of electromagnetic energy from a source such as a battery or electrical mains. Additionally or alternatively, the defogging unit may include a source (e.g. battery) of the electromagnetic energy. The defogging unit may further include connectors to deliver the electromagnetic energy to the heating unit. The delivery of the electromagnetic energy is preferably via a physical electrical connection with the heating unit such as by electrical wires, but it may be possible to transfer the electromagnetic energy by way of an inductive link (e.g. coaxially aligned transmitter coil and receiver coil) or other wireless electrical connection.

In some embodiments, the source of electromagnetic energy is located in the main body. Having the electromagnetic source located in the main body instead of e.g. in the head may offer advantages. Further, if the removable head needs to be replaced after having been used a number of times, the cost of the head may be kept low.

Electromagnetic energy, generally referred as electric power, may include energy contained in a DC, pulsating DC, or AC electric current or in electromagnetic radiation or in a static or time-varying electromagnetic field. Such energy may be provided by way of battery or mains supply.

The defogging unit may further include a control unit that is configured to control the operation of the heating unit. For example, the control unit may perform at least one of determining how much energy needs to be delivered to the heating unit, adjusting heat setting of the heating unit, switching off supply of electromagnetic energy, determining time for which the energy to be delivered or supplying energy for a specified time period. The control unit may further be configured to monitor the performance of the heating unit and/ or effectiveness of defogging. The control unit may also include a storage unit to store digital information such as monitoring results and may further to configured to generate a notification signal such as an audible alarm or visual indication through an LED of the scanning system when the monitoring result indicates performance below an acceptable level. The acceptable level may be predefined by way of a measurable parameter such as defogging time, or energy consumption, etc.

The heating unit is configured to convert electrical power to heat which is transferred to the secondary optical component in the sleeve by way of thermal conduction. For example, the heating unit includes a resistive element made of metal or resistive film heaters that may be printed on a thin substrate. The latter may be printed on a substrate like a flexible substrate and typically offers low profile form factor, improved temperature uniformity, quick thermal response due to low thermal mass, low energy consumption. Other types of heating units may also be considered.

In one embodiment, the heating unit includes a dedicated heater such as an embedded heater that is configured solely to generate heat prior and/ or during scanning of the patient's teeth. In another embodiment, the heating unit includes at least one component in the housing, the at least one component being configured to generate heat in response to the at least one component performing a scanning related function during scanning of the patient. The scanning related function is different from solely to generate heat. For example, the at least one component may be an illumination unit comprising a light source such as one or more LEDs that are configured to generate light, i.e. scanning related function, for illuminating an object to be scanned. However, in response to receiving electrical power to generate the light for illuminating the object, the light source also generates heat that is transferred, through the connection component, to the secondary optical component by way of thermal conduction during scanning. The heat generated by the at least one component during the scanning may be understood as a waste or residual heat that gets generated when the at least one component performs scanning related function during scanning. Other at least one component producing the waste or residual heat may include, but not limited to, the processor such as an Field-Programmable Gate Array (FPGA) and/ or ARM processor that is configured to generate processed data by processing one or more of the acquired data as the scanning related function, wireless (wifi) module that is configured to wirelessly transmit the processed data as the scanning related function, and image sensor configured to generate the plurality of images based on the incoming (reflected) light received from the illuminated dental object as the scanning related function.

In some embodiments, an electrically powered heating unit is located at the head, with power supplied from the remainder of the handheld 3D scanner, such as from the main body of the scanner.

The heating unit is usually connected to the connection component such that the generated heat is thermally conducted from the heating unit to the connection component. In an embodiment, the heating unit is arranged on the connection component. The heating unit may be arranged on the connection component by way of thermal adhesive, thermal paste, flexible conductive material or a mechanical spring force clamping parts together. Additionally or alternatively, the heating component may be arranged on the connection component by way of welding or heat conducting (e.g. metallic) mechanical fasteners. In another embodiment, the heating unit is arranged on the connection component comprises an embedded heating unit in the connection component. In another embodiment, the heating unit is physically connected to the connection component using a thermal conductive material such as a solid thermal conductive material. In other words, the heating unit may not be arranged on the connection component but is physically connected to the connection component using a thermal conductive material such as a solid thermal conductive material like metal wires or metal strips. For example, the heating unit may be arranged on the framework with a connector such as a sheet made of heat conducting material running along the framework connects the heating unit to the connection component. The heating unit may be arranged directly on the framework by way of a glue or other fastening means with the sheet at least partly covering the heating unit or the heating unit may be arranged on the framework with an intermediate material such as the sheet in between the heating unit and framework. In any of the disclosed embodiments, when the head is mounted on the mounting portion and the heating unit generates heat by application of the electrical power, the generated heat is transferred by thermal conduction to the connection component either directly or via the heat conducing connection (e.g. metal strips or wires). Thus, the connection component provides a thermal pathway allowing the generated heat to travel across the connection component from the heating unit to secondary optical component.

In an embodiment, the sleeve is removably attached with the housing such that coupling the sleeve with the housing arranges the secondary optical component in physical connection with the heating unit via the connection component for permitting thermal conduction of the generated heat from the heating unit to the secondary optical component. The coupling allows the sleeve to be in an immovable position in relation to the housing immediately prior or during scanning of the patient. The term removably attached refers to the sleeve being configured to be mounted on or dismounted from the housing. Typically, the removably attached includes removably attaching at least in part in a slidable manner such that the sleeve may be mounted on or dismounted from the head of the housing by way of a sliding motion of the sleeve over the housing. The sleeve may include an opening that is configured to receive the head of the housing. Thereafter, the sleeve may be at least in part slid over the over the housing such that the sleeve gets mechanically coupled with the housing, and the secondary aperture gets positioned in alignment with the primary aperture. "At least in part" refer to that the coupling may be achieved solely by the sliding motion or sliding motion in combination with other movement such as relative rotation of the sleeve and head. The alignment of the primary aperture and the secondary aperture with the secondary optical component arranged therein, allows the scanning signal to pass through the primary aperture, preferably having the primary optical component arranged therein, and secondary optical component. Thus, the alignment positions the signal receiving section of the secondary optical component in a relative position with respect to the primary aperture/ signal receiving section of the primary optical component such that the scanning signal may pass through the signal receiving section of the secondary optical component and primary aperture, or through the signal receiving section of the secondary optical component and signal receiving section of the primary optical component arranged at the primary aperture. Furthermore, the secondary optical component gets physically connected to the heating unit via the connection member, which is physically connected to the secondary optical component when the sleeve is coupled with the housing.

The term arranging the secondary optical component such as at the secondary aperture and/ or in relation to the housing when the sleeve is coupled with the housing may refer to at least one of physical arrangement, physical structure or material property that allows transfer of the generated heat to the secondary optical component through thermal conduction.

In one embodiment, arranging the secondary optical component to allow transfer of the generated heat from the heating unit to the secondary optical component through thermal conduction includes arranging a conductive material between the connection component and the secondary optical component. The conductive material may include a thermal adhesive or thermal paste or other conductive material. Additionally or alternatively, the secondary optical component may be arranged in a way to establish direct physical contact between the connection component and secondary optical component. Considering that the sleeve with the secondary optical component arranged therein is removably attached to the housing, the physical contact between the connection component and the secondary optical component is also releasable such as by applying pull-out force on the sleeve to dismount sleeve from the housing. The thermal conductivity of the arranged conductive material is higher than that of the arranged primary optical component. The arranged primary optical components refer to different embodiments describing "arranging the primary optical component", as disclosed earlier. The arranged conductive material is made up of a solid material typically having a thermal conductivity, e.g. higher than 1.5 Wm⁻¹K⁻¹ at room temperature.

Additionally or alternatively, arranging the secondary optical component to allow transfer of the generated heat from the heating unit to the secondary optical component through thermal conduction includes having the secondary optical component that is made up of a solid material having a high thermal conductivity. Thus, use of such material allow efficient transfer of the generated heat to the secondary optical component. The thermal conductivity of the secondary optical component is higher than that of the arranged primary optical component. The arranged primary optical components refer to different embodiments describing "arranging the primary optical component", as disclosed earlier. The secondary optical component is made up of a solid material having a thermal conductivity typically higher than 10 Wm⁻¹K⁻¹ at room temperature. The material having the high thermal conductivity for the secondary optical component is typically selected from mineral family of corundum such as Sapphire glass.

Additionally or alternatively, arranging the secondary optical component to allow transfer of the generated heat from the heating unit to the secondary optical component through thermal conduction includes the secondary optical component including a substrate, which may be the secondary optical component itself, having a deposit of high thermal conductivity disposed on the substrate such as at least one of a secondary first surface or a secondary second surface of the secondary optical component. Thus, use of such deposit allows the generated heat to be transferred over the surface of the secondary optical component, thus heating up the secondary optical component. The thermal conductivity of the deposit material is higher than that of the arranged primary optical component. The arranged primary optical components refer to different embodiments describing "arranging the primary optical component", as disclosed earlier. The substrate may be selected from selected from mineral family of corundum such as Sapphire glass. The deposit material and/ or substrate may have a thermal conductivity typically higher than 10 Wm⁻¹K⁻¹ at room temperature.

Additionally or alternatively, arranging the secondary optical component to allow transfer of the generated heat from the heating unit to the secondary optical component through thermal conduction includes the secondary optical component including a multi-layered structure where at least two layers, which may be a deposit layer, of the multi-layered structure have different thermal conductivity and/ or optical properties. The multi-layered structure includes a plurality of layers that are arranged over one another, i.e. overlaid, along a propagation direction of the scanning signal and/ or a plurality of layers that are arranged adjacent to one another along a surficial dimensions of the secondary optical component. It is preferred that all the layers in the secondary optical component are made of materials having high thermal conductivities. However, in some scenarios some of the layers such as an overlaid layer sandwiched between two layers may not have high thermal conductivity. Thus, in some embodiments, at least one or preferably at least two such as the outermost layers of the multi-layered secondary optical components have high thermal conductivities. The outermost layers may correspond to layers having the secondary first surface and secondary second surface. This may be particularly useful if the secondary optical component is required to have a specific thermal and/ or optical properties while still allowing heat transfer by thermal conduction to the primary optical component. The thermal conductivity of the layers having high thermal conductivity includes thermal conductivity that is higher than that of the arranged primary optical component. The arranged primary optical components refer to different embodiments describing "arranging the primary optical component", as disclosed earlier.

In any of the above embodiments, the layers may be a deposit material and/ or a layer with a predefined thickness.

The secondary optical component includes optical properties that allow the scanning signal to pass through the secondary optical component such that the oral cavity may get illuminated and the sensor may receive the light reflected from the oral cavity in response to the illumination. Typically, the secondary optical component is a transparent glass or any other material that allows transmission of the scanning signal without substantial alteration to the scanning signal to avoid errors in acquired data or acquired images. The term "substantial alteration" may refer to enough alteration in the scanning signal to degrade the acquired data or acquired two-dimensional image quality, and the accuracy of the digital 3D representation. The secondary optical component includes the secondary first surface or the secondary second surface, wherein the secondary first surface and the secondary second surface are opposite surfaces of the secondary optical component with one surface facing the environment such as oral cavity environment when sleeve is inserted in the oral cavity and the other surface facing the primary optical component when the sleeve is coupled with the housing.

When scanning a bodily orifice such as the oral cavity of a patient using the optical scanning system, the parts such as surface exposed to the environment in the bodily orifice is disinfected and preferably to sterilized in order to avoid infections and the transfer of a potential disease from one patient to the another. For the disclosed optical scanning system such as the intraoral 3D scanner, the sleeve therefore may either be replaced by a new sleeve, i.e. the sleeve is removably attached and disposable or an already used sleeve is disinfected, if not sterilized, prior to scanning a new patient with the same sleeve, i.e. the sleeve is removably attached and reusable.

The sleeve is made up of at least in part with a non-conductive material such as a plastic material (for example, by injection molding). For the reusable sleeve, in an embodiment, it may be an advantage that the sleeve may be dismounted from the housing and that the sleeve may withstand sterilization in an autoclave, since this would allow the sleeve to be removed after scanning and to be sterilized alone without the need for exposing the entire optical scanning system (i.e. intraoral 3D scanner) to a sterilization procedure. After sterilization, the sleeve may be mounted on the housing and be reused for scanning the next patient. In some embodiments, the sleeve is configured to withstanding sterilization in an autoclave at least partly by the choice of materials. The sleeve may be manufactured in a material, such as thermoplastic like PSU, which is capable to withstand autoclave treatment. If a heat conducting element of the sleeve comprises a heat conductive layer which may not withstand the harsh treatment of an autoclave sterilization process it may be protected by a protective layer of the heat conducting element, such as a protective layer made in stainless steel.

In an embodiment, the sleeve includes a secondary optical component arranged at the secondary aperture. The secondary optical component arranged at the secondary aperture is configured to receive the generated heat from the heating unit by way of thermal conduction. The term arranging the secondary optical component may refer to at least one of physical arrangement, physical structure or material property that allows transfer of the generated heat to the secondary optical component. The secondary optical component is permanently arranged or removably arranged at the secondary aperture, wherein the arranging includes attaching the secondary optical component at the secondary aperture. The removably arranged secondary optical component allows for replacing a defective secondary optical component.

The sleeve includes the secondary aperture where the secondary optical component is arranged. The secondary optical component may be attached to the sleeve by using a glue such as by way of thermal adhesive or thermal paste. Another method of attaching the secondary optical component to the sleeve is to weld a thin sheet made of some other material, e.g., metal to the plastic material and use glue to bond the secondary optical component to the sheet. There is a much wider choice of autoclavable glues that bond metal to glass. In yet another embodiment, the sleeve includes a secondary frame comprising the secondary optical component. The secondary frame holds the secondary optical component and is configured to physically connect with the connection member of the housing when the sleeve is coupled with the housing. The secondary frame includes a structure at least partially surrounding and supporting the secondary optical component. As an example, the structure may include at least one of head, jambs, or sill like a window frame. As another example, the structure may include one or more clamps that supports the secondary optical component. Other form of structures may also be used and within the scope of the disclosure. The secondary frame may further include an engagement element that is configured to physically interact with the housing such that the secondary optical component is brought in physical connection with the connection component of the housing.

The secondary frame is usually made up of solid material including metal such as Aluminum but other solid material like metals or alloys thereof having high thermal conductivity may also be used. The solid material includes a thermal conductivity that is higher than that of the arranged primary optical component. The arranged primary optical components refer to different embodiments describing "arranging the primary optical component", as disclosed earlier. The solid material having a thermal conductivity higher than 7 Wm-1K-1 at room temperature. Thus, the secondary frame allows to establish a physical connection between the connection component and the secondary optical component. In an embodiment, the secondary frame may be provided with an anodization layer such as a black anodization layer that is configured to avoid stray light inside the scanner. The anodization layer, typically a thin layer, may have a low thermal conductivity, but the secondary frame that is made up of a solid material having a thermal conductivity allows for efficient transfer of the generated heat from the connection component to the secondary optical component. Thickness of the anodization layer may vary typically in the range of 0.5 µm to 100 µm.

In an embodiment, a relative position of the primary optical component and secondary optical component defines a gap therebetween when the sleeve is coupled with the housing. The gap comprises a gap-width that allows thermal insulation between the primary optical component and secondary optical component. Thus, the gap width allows for inhibiting such as preventing transfer of heat between the optical components such as from the secondary optical component to the primary optical component such as by radiation or convection across the gap width. Considering that the primary optical component may be inhibited from such as prevented from receiving the generated heat, the thermal insulation by way of the gap width may inhibit heat transfer of heat to the secondary optical component by radiation or convection across the gap width. There may still be some heat getting transferred via radiation or convection across the components, the defogging of the secondary optical component such as immediately prior to the scanning relies primarily on transfer of the generated heat from the heating unit to the secondary optical component via thermal conduction through the connection component rather than on from a heated up primary optical component to the secondary optical component across the gap width via convection or radiation. In principle, for the defogging of the secondary optical component, a larger gap width is usually preferable. However, the skilled person would appreciate that at least a part of the head and at least a part of the sleeve including the secondary optical component need to be inserted into the bodily orifice. Therefore, there is a practical limitation to how big the gap width may be. Nevertheless, the skilled person would acknowledge that gap width may also influence the effectiveness of the disclosed defogging system, i.e. the greater the gap width, the higher the effectiveness of the intraoral scanner comprising the disclosed defogging system. The gap width is generally between 0.1 to 1 mm, preferably less than 0.5 mm such as 0.2 mm to produce an effective defogging of the secondary optical component.

The term defogging system may include components and/ or arrangements that functionally operate to defog the insertable surfaces (e.g. optical components). This may include one or more of defogging unit, connection component, primary optical component secondary optical component, gap width, etc.

In an embodiment, the gap width is at least partially filled with a thermal insulating material. This may include air, vacuum, gas, a gel, transparent solid insulating material. The choice of the insulating material is based on non-interference with the scanning signal. This may be based on matching, as closely as possible, the refractive index of the insulating material (i.e. index matching material) with that of the primary optical component and/ or secondary optical element. In other words, optical property of the insulating material allows the scanning signal to pass through the insulating without any detrimental effect on the acquired data. For example, the optical property of the insulating material prevents the scanning signal to get altered or substantially altered when the scanning signal passes through the insulating material.

The disclosed technique for defogging of the secondary optical component such as immediately prior to or during the scanning relies on several factors, with each factor contributing to increasing the effectiveness of the defogging system. A combination of two or more factors may increase the effectiveness further compared a single factor in isolation. Some of the factors include
a) transfer of generated heat from the heating unit to the secondary optical component through thermal conduction. Direct transfer, through thermal conduction, of the generated heat across physically connected components (i.e. heating unit and secondary optical component) improves defogging such as in terms of reduced defogging time; and/ or
b) inhibiting such as preventing (e.g. avoiding), transfer of the generated heat from the heating unit to the primary optical component. Inhibiting such as preventing the transfer of generated heat ensures that the generated heat is not wasted/ lost on heating the primary optical component and may be focused on heating the secondary optical component. Nevertheless, as discussed earlier, it may be beneficial to have the primary optical component with high heat capacity, thus allowing the primary optical component to retain the heat for a long time (e.g. during the scanning); and/ or
c) inhibiting such as preventing (e.g. avoiding), transfer of heat between the primary optical component and the secondary optical component across a gap between the primary optical component and secondary optical component. As the secondary optical component heats through transfer of heat by thermal conduction, inhibiting such as preventing transfer of heat across the gap allows for the heat not getting transferred from the heated-up secondary optical component to the primary optical component. Thus, the generated heat is not wasted/ lost on heating the primary optical component. Nevertheless, as discussed earlier, it may be beneficial to have the primary optical component with high heat capacity, thus allowing the primary optical component to retain the heat for a long time (e.g. during the scanning).

It is to be noted that heat transfer occurs at a lower rate in materials of low thermal conductivity than in materials having high thermal conductivity. Therefore, to comply with above-listed factors a) and b), a relative thermal conductivity criterion may be defined. The criterion includes the materials defining a thermal path from the heating unit to the secondary optical component have higher thermal conductivity in comparison to materials defining a thermal path from the heating unit to the primary optical component. In another words, the thermal conductivity of a secondary thermal path defined between the heating unit and the secondary optical component is higher than the thermal conductivity of a primary thermal path defined between the heating unit and the primary optical component. The secondary thermal path is defined by materials (typically including the secondary optical component as well) used between the heating unit and the secondary optical component, and the primary thermal path is defined by materials (typically including the primary optical component as well) used between the heating unit and primary optical component. It may be preferred that thermal conductivity of each material defining the secondary thermal path is higher the thermal conductivity of each material defining the primary thermal path. However, it may not be necessary to comply with this thermal conductivity requirement at each material level, so long as the relative thermal conductivity criterion for the paths is satisfied. It may be noted that the disclosure provides specific ranges for thermal conductivities for different materials. However, the skilled person would appreciate that deviation from the disclosed ranges is within the scope of the disclosure so long as thermal conductivities of the secondary thermal path and primary thermal path complies with the relative thermal conductivity criterion.

In an embodiment, the head includes a primary engagement element and the sleeve includes a secondary engagement element. The primary engagement element and secondary engagement element are configured to physically interact during coupling of the sleeve with the housing. Such physical interaction brings the connection component and secondary optical component/ the secondary frame comprising the secondary optical component in a physical connection with each other. Even when the physical interaction physically connects the secondary frame with the housing, a physical connection is established between the housing (such as connection component) and secondary optical component because the secondary frame includes the secondary optical component. The primary engagement element and the secondary engagement element may be the same elements that are configured to couple the sleeve and housing, thereby providing the functions of coupling the sleeve and housing and bringing the connection component and secondary optical component/ the secondary frame in physical connection. Alternatively, the primary engagement element and the secondary engagement element may be different from the elements that allow coupling of the sleeve and housing, thereby only providing the function of bringing the connection component and secondary optical component/ the secondary frame in physical connection. The coupling refers to the sleeve attached to the housing such that the sleeve is in an immovable relation with respect to the housing immediately prior or during scanning of the patient.

In an embodiment, the head includes the primary engagement element and the sleeve includes the secondary engagement element. The primary engagement element and secondary engagement element are configured to physically interact. The physical interaction allows for keeping the connection component and secondary optical component/ the secondary frame comprising the secondary optical component in a physical connection with each other when the sleeve is coupled with the housing. Even when the physical interaction physically connects the secondary frame with the housing, a physical connection is established between the housing (such as connection component) and secondary optical component because the secondary frame includes the secondary optical component. The primary engagement element and the secondary engagement element may be the same elements that couple the sleeve and housing, thereby providing the functions of coupling the sleeve and housing and keeping the connection component and secondary optical component/ the secondary frame in physical connection. Alternatively, the primary engagement element and the secondary engagement element may be different from the elements that allow coupling of the sleeve and housing, thereby only providing the function of bringing the connection component and secondary optical component/ the secondary frame in physical connection. The coupling refers to the sleeve attached to the housing such that the sleeve is in an immovable relation with respect to the housing immediately prior or during scanning of the patient.

In an embodiment, one of the primary engagement element or secondary engagement element comprises a protrusion and another of the primary engagement element or secondary engagement element comprises a surface that is configured to physically interact with the protrusion. The term protrusion may refer to an element on a surface extending out relative to the surface or the entire surface being a non-planar surface like convex surface. If the protrusion is on the sleeve, then the protrusion extends towards the volumetric space of the housing. Whereas, if the protrusion is on the head, then the protrusion extends away from the volumetric space of the housing. The protrusion and the surface are configured to physically interact to bias the secondary optical element/ secondary frame comprising the secondary optical element and connection component towards each other. The physical interaction generates a force that biases the secondary optical element/ secondary frame comprising the secondary optical element and connection component towards each other. The force is typically applied during coupling of the sleeve with the housing but may be continuously applied when the sleeve is coupled with the housing. As an example, the protrusion is configured to apply a force on the surface such that secondary optical element/ secondary frame comprising the secondary optical element and connection component are biased towards each other.

In one embodiment, an inner surface of the sleeve includes the protrusion facing towards an outer surface of the head, the outer surface (i.e. interacting surface) being configured to physical interact with the protrusion. In another embodiment, an outer surface of the head includes the protrusion facing towards an inner surface of the sleeve, the inner surface (i.e. interacting surface) being configured to physical interact with the protrusion. In any of the preceding embodiments, the protrusion is positioned such that during the coupling, the physical interaction between the protrusion and the interacting surface generates a force that pushes (i.e. biases towards) the connection component in a direction towards the secondary optical component/ secondary frame and/ or pushes (i.e. biases towards) the secondary optical component/ secondary frame in a direction towards the connection component. The force results in establishing and/ or maintaining the physical connection between the connection component and secondary optical component/ secondary frame, as defined by a contact surface area at an interface.

In another embodiment, the secondary optical component is mounted in the sleeve by using an elastic component such as an elastic glue (e.g. elastic silicone glue) or a gasket, which may have a shore value above A10 such as between A10 to A80. The elastic component may facilitate in securing the position of the secondary optical component in the sleeve. During the coupling between the secondary optical element and the connection component and/ or when the sleeve is coupled with the housing, the secondary optical component is arranged such that the connection component faces one surface of the secondary optical component and the elastic component faces another surface of the secondary optical component. The connection component is configured to apply a force on the secondary optical component in one direction (e.g. downward direction), thus compressing the elastic component. In response to this force applied on the secondary optical component, the elastic component is configured to apply a responsive force in an opposite direction (e.g. upward direction). The interaction between applied force and responsive force ensures that an optimal contact between the connection component and the secondary optical component upon mounting of the sleeve on the housing is achieved. The skilled person would appreciate that the nature of the elastic component that is configured to provide a responsive spring force ensures that the responsive force is adjusted in accordance with the applied force so that the secondary optical component is not subjected to an excessive force. The optimum force by way of the applied force and responsive force of the elastic component may also provide a mechanical seal between the connection component and the secondary optical component. Also, this embodiment may be used in combination with the earlier disclosed embodiment of using protrusion for producing biasing force, which may represent the applied force.

In an embodiment, one of the primary engagement element or secondary engagement element comprises a guide and another of the primary engagement element or secondary engagement element comprises a guide channel that is configured to receive (i.e. physically interact with) the guide. The guide channel usually includes an entrance through which the guide enters the guide channel. The guide is configured to move along the guide channel and moving the guide along the guide channel is configured to bias the secondary optical element/ secondary frame comprising the secondary optical element and connection component towards each other. Reference to a guide or a guide channel is intended to include the plural form as well, i.e. to have the meaning "at least one". It is understandable that a guide will be received in a corresponding guide channel.

In one embodiment, the guide comprises at least one edge of the secondary optical component/ at least one edge of the secondary frame and the connection component comprises the guide channel. In another embodiment, the guide comprises at least one edge of the connection component and the secondary optical component/ secondary frame comprises the guide channel. In any of the preceding embodiments, the guide channel includes an elongated groove along which the at least one edge may be guided and moved from the entrance until a termination point along the groove length, thus bringing into position (i.e. biased towards) the secondary optical element/ secondary frame comprising the secondary optical element and connection component towards each other. Bringing the guide in position within the guide channel results in establishing and/ or maintaining the physical connection between the connection component and secondary optical component/ secondary frame, as defined by a contact surface area at an interface such as at the interface of the guide and guide channel.

In an embodiment, the physical connection between the connection component and secondary optical component/ the secondary frame comprising the secondary optical component is defined by a contact surface area at an interface between the connection component and secondary optical component/ the secondary frame. The interface corresponds to physical contact between the interfacing components, i.e. connection component and the secondary optical component/ secondary frame. Accordingly, the contact surface area corresponds to the area of portions of interfacing components that are in physical contact with each other.

It is apparent that the efficiency of thermal conduction from the connection component to secondary optical component/ the secondary frame would increase with an increase in the contact surface area. The contact surface area may be increased at most until the contact surface area starts interfering with a signal receiving section of the secondary optical component. In other words, a portion of the connection component interfacing the secondary optical component/ the secondary frame comprising the secondary optical component is dimensioned such that when the sleeve is coupled with the housing, the connection component is prevented from interfering with the signal receiving section of the secondary optical component.

Thus, the portion of the connection component may be dimensioned between a lower threshold value and a higher threshold value. The lower threshold value may be defined by a dimension that satisfies a predetermined criterion such as minimum contact surface area, or defogging time, etc. The higher threshold value may be defined by a dimension resulting in the portion starting to interfere with the signal receiving section of the secondary optical component. The signal receiving section may be understood as a portion of the optical component through which the scanning signal is configured to pass through or passes through for the purpose of scanning the patient.

Apart from the contact surface area, the efficiency of the thermal conduction may be increased by other factors. A first exemplary factor includes contact pressure between the connection component and the secondary optical component/ secondary frame comprising the secondary optical component to maintain a satisfactory contact between the components for achieving an effective thermal conduction. This may be achieved by having the engagement element such as a spring based engagement element configured to apply a force on the connection component and/ or secondary optical component/ secondary frame to maintain the physical connection between the interfacing components. A second exemplary factor include increasing the efficiency of the thermal conduction by way of modifying surface roughness, waviness and flatness of interfacing portion of one or more of the connection component, secondary optical component or secondary frame comprising the secondary optical component such as by use of the deposit material. For example, Average Surface roughness of the connection component at interface(s) to conduct heat is typically kept low and may be in the range of 0.100 - 3.00 µm such as around 2.00 µm. Additionally of alternatively, Maximum Surface roughness (Rm) of the connection component at interface(s) to conduct heat is typically kept low and may be below 12 µm, such as below preferably below 10 µm, more preferably below 8 µm, even more preferably below 6 µm, most preferably below 4 µm. Although it may be preferred to keep both Maximum Surface roughness and the Average Surface Average low, it is generally preferred to use the Maximum Surface roughness as a more relevant criterion. For example, a connection component having a lower Maximum Surface roughness is usually preferred over connection components having same or lower Average Surface roughness but a higher Maximum Surface roughness. Other factors influencing the efficiency of thermal conduction from the connection component to the secondary optical component/ secondary frame comprising the secondary optical component may also be considered.

In an embodiment, the secondary optical component comprises layers of coating of Ta₂O₅ and SiO₂ on at least one surface of the secondary optical component. The secondary optical component comprises hydrophobic coating of Perfluorodecyltrichlorosilane (FDTS) over the layers of coating. An intermediate bonding layer of Al₂O₃ is typically sandwiched between the hydrophobic coating and coated layers.

In an embodiment, the head of the housing may include a temperature sensor. The temperature sensor may be enclosed within the housing. The temperature sensor is arranged proximal to such as adjacent to the primary aperture. The temperature sensor is configured to measure temperature in the head, and provide sensor measurements as signal to a control unit. The control unit, based on the received sensor measurement signal, is configured to provide feedback control signal. The control unit provides the feedback control signal at least when the sensor measurement signal is beyond a threshold temperature value. The threshold temperature value may be predefined based on a permissible temperature in the oral cavity, i.e. a predefined threshold temperature value may represent a value that ensures that the temperature in the oral cavity during scanning does not exceed the permissible temperature. In an embodiment, the feedback control signal is configured to put the scanner in a throttling mode. The throttling mode includes a scanner operation mode where the speed of data acquisition or data transmission is limited, or even stopped. The data acquisition may refer to acquiring data in response to illumination of a dental object, and data transmission may refer to transmitting acquired data or processed data comprising processed acquired data from the scanner to a remote processor. Putting the scanner in the throttling mode allows the scanner to cool down. This allows the temperature in the head to lower at or below the threshold temperature value, thereby lowering the temperature in the oral cavity at or below the predefined permissible temperature. Additionally or alternatively, the feedback control signal is configured to control the temperature setting of the embedded heating unit such that the sensor measurement at the temperature sensor is at or within the threshold temperature value.

In another embdodiment, the system includes a mounting sensor to detect whether the sleeve is in a mounted state onto the housing. The mounting sensor may include an optical sensor such as an RGB sensor, electrical connection-based sensors, mechanical coupling sensors comprising locking mechanisms. The intraoral scanner may be used for scanning only after the sleeve is determined to be in a mounted state.

In an embodiment, when the sleeve is coupled with the housing, a part of the connection component interfacing the secondary optical component/ the secondary frame comprising the secondary optical component is arranged in relation to the secondary optical component/ the secondary frame to avoid the connection component interfering with a signal receiving section of the secondary optical component. This may be achieved by having an optimum dimension of the portion of the connection component, as described earlier.

### Brief description of the drawings

The embodiments of the disclosure, together with its advantages, may be best understood from the following illustrative and non-limiting detailed description taken in conjunction with the accompanying figures in which
Fig. 1 illustrates an optical scanning system such as a 3D intraoral scanner system according to an embodiment;
Fig. 2a illustrates a defogging system from a first view according to an embodiment;
Fig. 2b illustrates defogging system from a second view according to an embodiment;
Fig. 3a illustrates a cross sectional view from a first perspective such as front view of the head and sleeve fully mounted on the housing according to an embodiment;
Fig. 3b illustrates a cross sectional view from a first perspective such as front view of the head and sleeve fully mounted on the housing according to an embodiment;
Fig. 4a illustrates, from a first view such as side view, a first stage during mounting of the sleeve onto the housing according to an embodiment;
Fig. 4b illustrates, from the first view such as side view, a second stage during mounting of the sleeve onto the housing according to an embodiment;
Fig. 4c illustrates, from the first view such as side view, a third stage during mounting of the sleeve onto the housing according to an embodiment;
Fig. 5 illustrates, from the second view such as bottom view, the first stage during mounting of the sleeve onto the housing according to an embodiment;
Fig. 6 illustrates a zoomed cross-sectional view of the head and sleeve fully mounted on the housing according to an embodiment;
Fig. 7a illustrates the primary optical component according to an embodiment;
Fig. 7b illustrates the primary optical component according to an embodiment;
Fig. 7c illustrates the secondary optical component according to an embodiment;
Fig. 7d illustrates the secondary optical component according to an embodiment;
Fig. 8 illustrates the secondary component according to an embodiment; and
Fig. 9 that illustrates a multilayered secondary component according to an embodiment.

### Detailed Description

In the following description, reference is made to the accompanying figures that show by way of non-limiting illustration how the disclosed method or system may be implemented.

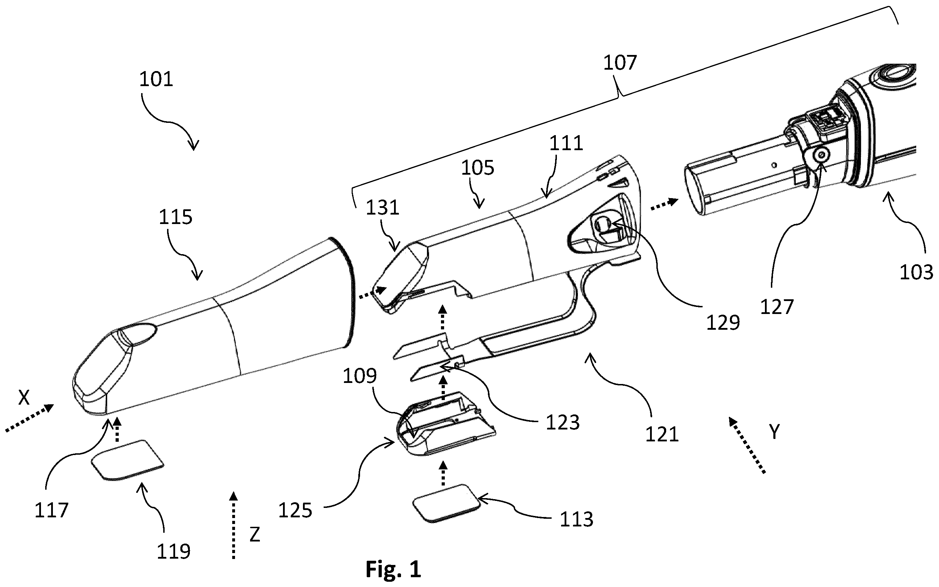

Fig. 1 illustrates an optical scanning system such as a 3D intraoral scanner system according to an embodiment. The 3D intraoral scanner system 101 includes an intraoral scanner that includes a main body 103 enclosing at least the optical components that are configured to operate during a 3D scanning of the dental object such as oral cavity. The 3D scanning refers to the process of acquiring the plurality of two-dimensional images of the dental object based on which three-dimensional digital representation of the dental object is generated. The main body may further include one or more electronic components that are also configured to operate during 3D scanning of the dental object. The exterior surface of the main body is formed in plastic shells that is easy to clean, thus offering a hygienic solution. The intraoral scanner is designed to be held like a pen, i.e. operate in a pen grip during the 3D scanning or may include a handle grip, which is held during the 3D scanning.

Irrespective of the grip, the intraoral scanner is configured to be moved over the dental object such as the oral activity during the 3D scanning. The scanner may be a focus scanner, OCT scanner, triangulation scanner, confocal scanner or any kind of imaging apparatus capable of acquiring two-dimensional images that may be processed into 3D information such as three-dimensional digital representation. The illustrated Fig. 1 shows a handheld intraoral scanner system 101 which is a focus scanner that is configured to acquire a sequence of 2D images while moving focus optics and illuminating a pattern onto the object to be scanned. Typically, the movement of the focus optical and illuminating a pattern onto the object occurs simultaneously. The sequence of 2D images is processed into the 3D information. The 3D information may be transmitted to a scanning application software. The intraoral scanner system 101 may use an optical system where the illumination probe light and imaging probe light (i.e. reflected light) are utilizing the same path through the main part of optical system inside the main body. The intraoral scanner includes a distal end that is shaped like an elongated probe head 105 that is configured to be inserted into the oral cavity and to direct the probe light to, and reflected light from the dental object to be scanned. The housing 107 includes the head 105 and the main body 103. The head is configured to be attached with the rest of the housing, e.g. at a mounting portion of the housing. The mounting portion 127 may include a locking unit that is configured to engage with a complimentary locking unit 129 of the head to allow locking the head with the mounting portion.

The head 105 may include a mirror or prism arranged at its distal end for guiding the probe light to and reflected light back from the dental object to be scanned. The probe light (i.e. illumination signal) propagates through the framework 111 of the head 105 and reflects off the mirror and across the primary aperture 109 towards the dental object. Similarly, the reflected light from the dental object enters the head by travelling across the primary aperture 109 and propagates through the framework 111 after getting reflected from the mirror. Due to hygienic reasons, the primary aperture 109 may be sealed from the external environment. This is typically achieved by arranging the primary optical component 113 at the primary aperture. The disclosed arrangement allows for the scanning signal to be transmitted through the primary aperture 109 while maintaining a microbial barrier to the exterior of the head enclosure. The framework 111 may be manufactured by injection molding of high-performance thermoplastics like Polysulfones (PSU).

The scanner head 105 may additionally be configured to receive a sleeve 115 or sheath, which can be detachably mounted to the scanner housing 105. The sleeve is configured to at least partially cover the head 105. The sleeve 115 may have a similar shape as the head 105 and may contain a second aperture 117 which is configured to be aligned with the primary aperture 109 when the sleeve 115 is securely positioned such as mounted on the housing 105. The secondary aperture 117 is configured to contain a secondary optical component 119, which is configured to allow the probe light to be transmitted without substantially alteration of characteristics of the probe light, such as the polarization.

To restrict fogging of the secondary optical component 119 when scanning inside humid environment such as the human oral cavity, the head unit includes a defogging unit 121. Fogging may cause condensation at the secondary optical component 119 and this may deteriorate the quality of transmission of the scanning signal (e.g. illumination light and/ or reflected light), leading to loss in data acquisition quality. The defogging unit 121 is constructed to avoid fogging of the secondary optical component 119 by raising the temperature of the secondary optical component 119 to a temperature above the dew point of the intended scanning environment. In case of scanning the human oral cavity, the target temperature is typically approximately in the range of 30°C to 40°C.

The defogging unit 121 may include a heating unit 123 for converting electricity into heat. In the disclosed embodiment of Fig. 1, the heating unit is embedded into a printed circuit board (PCB) which may establish electrical connection between the main body 103 and heating unit 123, thereby controlling the heating. In one example the defogging unit is located at the connection component 125. The connection component 125 may be multi-functional as it is shaped as an insert frame part which is designed to be held at the framework 111 and simultaneously is configured to interface both with the heating unit 123 and the primary optical component 113, which is arranged at the connection component.

One functionality of the connection component 125 is to transfer the generated heat, via thermal conduction, from the heating unit 123 to the secondary optical element 119 as effectively as possible. The connection component 125 may be fabricated from a material with an intrinsic high thermal conductivity, such as a metal like aluminum (Al) like Alu 6082 T6 or an alloy or any other composite material. Alternatively, the connection component 125 may include a structure facilitating effective heat transfer to the secondary optical component 119. For example, the connection component 125 may include a plastic frame comprising one or more sheets or layers of a super conducting material such like sheets of armchair graphene nanoribbons providing thermal pathways (e.g. heat guides) for conducting the generated heat from the heating unit to the secondary optical component 119. The thermal pathways may be coated with epoxy such that the pathways are sandwiched between the frame and coating. The connection component 125 usually extends towards the secondary optical component 119 such that a physical contact between the heating unit 123, through the connection component 125, and secondary optical element 119 may be established. This allows the thermally conducting the generated to the secondary optical component and raising the temperature of the secondary optical component.

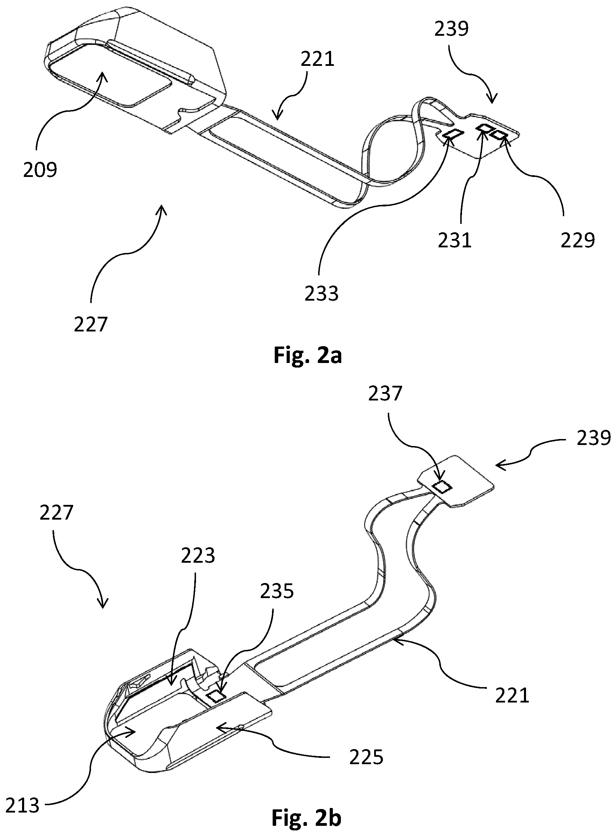

Fig. 2a illustrates a defogging system from a first view according to an embodiment. Fig. 2b illustrates defogging system from a second view according to an embodiment. The defogging system 227 includes the defogging unit 221 and connection component 225. The defogging unit 221 is shaped in a symmetric forklike flexible printed circuit board (PCB), where each of two side wings of the PCB contain a heating unit 223. The heating unit 223 includes a resistive heater traces made of thin long copper wires. To ensure an optimal thermal coupling between the heating unit 223 and the connection component 225, any cover of the PCB may be removed in those interface sections that shall be heated. The heating unit 223 may be attached to the aluminum wall of the connection component 225 by means of Pressure-sensitive adhesive (PSA) which may also provide an acceptable thermal coupling between the parts.

The defogging unit 221may include a flexible PCB that may have a minimum bend radius of 2.5m. The PCB may be colored black and designed by keeping components out of the optical path to avoid any straylight arising inside the framework during scanning. The PCB may contain an Inter-Integrated Circuit (I²C) and/ or Pulse Width Modulation (PWM) communication 229 interface and a safety cut-off circuit 231 a EEPROM memory 233 to store information such as 2kb AT24C02C EEPROM with an 8 x 256 memory organization. The region close or proximal to the embedded heating unit may include a temperature sensor 235 that is configured to provide signal, representing sensor measurements, to a control unit that is configured to provide feedback control of the temperature based on the signal. The feedback control of the temperature based on the signal allows the control unit to ensure that the temperature setting on the embedded heater is controlled such that the heat at the secondary optical component is maintained to produce necessary defogging, without overheating. Additionally or separately, the heating unit may also contain an optical sensor 237 such as an RGB color sensor located proximal to a connector interface 239 that is configured to provide electrical connection of the heating unit with the housing 103. The optical sensor is configured to monitor color changes in response to positioning of the sleeve on the housing. The optical sensor may perform monitoring by detecting color (e.g. color change) through a small optically transparent window in the head 105. The optical sensor is configured to provide signal, representing color change, to a control unit. The control unit is configured to determine whether the sleeve 115 is correctly mounted onto the housing 107 based on the signal. For example, the color sensor 237 white LED may operate in a pulse mode to check if a certain color is present or if something is simply reflecting the generated light back to the sensor 237. In this case it may be concluded that a sleeve 115 is present. The intraoral scanner may be used for scanning only after the sleeve is determined to be correctly mounted. The skilled person would appreciate that there are other ways of determining correct mounting of the sleeve onto the housing. For example, the signal may be based on establishing an electrical connection between complimentary contact points of the housing and sleeve. Establishing the electrical connection provides the signal to the control unit that determines whether the mounting is proper based on the signal. Another example includes the signal may be based on establishing an physical connection between complimentary locking points (e.g. click locking mechanism) of the housing and sleeve. Establishing the physical connection may act as a trigger event that sends a signal to the control unit that determines whether the mounting is proper based on the signal. Other variations are within the scope of the disclosure. 209 represents primary aperture and 213 represents primary optical component arranged at the primary aperture 209.

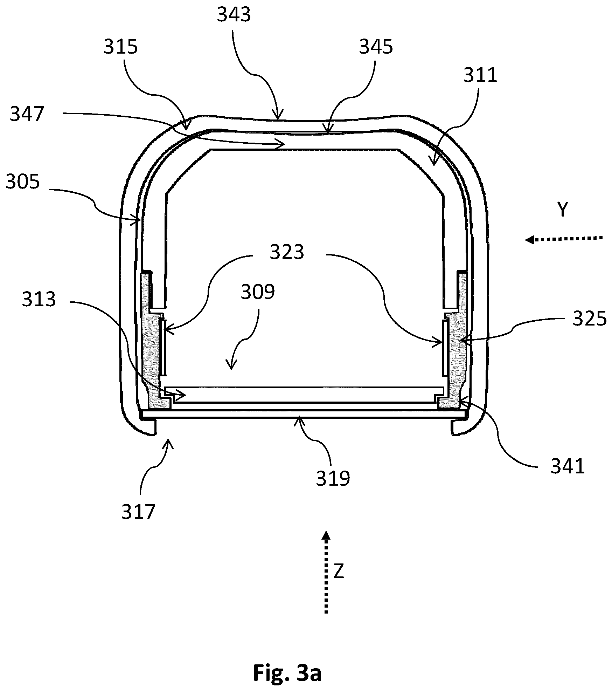

Fig. 3a illustrates a cross sectional view from a first perspective such as front view of the head and sleeve fully mounted on the housing according to an embodiment. The front view is in the direction X (see Fig. 1), through the distal section of the head 305 of the intraoral scanner system 101 when the sleeve 315 is fully mounted, i.e. in position, onto the housing 107 with the primary aperture 309 and secondary apertures 317 in alignment with each other. It is preferred to achieve a good heat transfer from the heating unit 323 to the secondary optical component 319. This is achieved by establishing a reliable physical contact between the secondary optical component 319 and connection component 325 when the sleeve 315 is fully mounted onto the housing 107. A part of the connection component 325 surrounding the primary optical component 313 may be shaped, e.g. like narrow rectangular bars, to extend downwards away from the primary optical component 313. The term downwards denotes a direction opposite to direction Z (see Fig. 1). The protruding part may be designed as a contact interface 341 for establishing contact with the secondary optical component 319 when the sleeve is positioned such as slid onto the head 305. A pocket 343 at the top part material surface of the sleeve 315 may generate a primary engagement element 345 in the form of a protrusion on the inside of the surface. The term top denotes a direction in the direction Z (see Fig. 1). The protrusion 345 at the top part of the sleeve 315 creates a contact force such as a spring force applied on the secondary engagement element 347 at the framework 311 of the head 305 when the sleeve 315 is fully mounted on the housing. The secondary engagement element 347 may respond by applying an equal spring force in the opposite direction. This reaction force pushes the secondary optical component 319 situated in the sleeve 315 in physical contact with the contact interface 341. The part of the contact interface 341 initially receiving the secondary optical component 319 is shaped like longitudinal runners or skis similar to the structure on a sled. The force applied on the connection component 325 and the secondary optical component 315 because of the interaction between the primary engagement element 345 and secondary engagement elements 347 provides enough contact force between the connection component 325 and the secondary optical component 315 to ensure a good physical connection for thermal conduction between connection component 325 and the secondary optical component 315. The contact force is usually along the Z-direction (see Fig. 1) and may be in the range of 1 to 24 N. The weight of the connection component is kept low, for example below 1.75 g, preferably below 1.5 g, more preferably below 1.25 g such as 1.2 grams. Surface roughness of the connection component 325 such as the profile roughness parameter (Ra) at interface(s) to conduct heat is usually kept as low as possible. There may be manufacturing limitation that affects how low profile roughness parameter (Ra) value may be achieved. The profile roughness parameter (Ra) may be in the range of 0.100 - 3.00 µm such as around 2.00 µm. Additionally of alternatively, Maximum Surface roughness (Rm) of the connection component at interface(s) to conduct heat is typically kept low and may be below 12 µm, such as below preferably below 10 µm, more preferably below 8 µm, even more preferably below 6 µm, most preferably below 4 µm. Although it may be preferred to keep both Maximum Surface roughness and the Average Surface Average low, it is generally preferred to use the Maximum Surface roughness as a more relevant criterion.

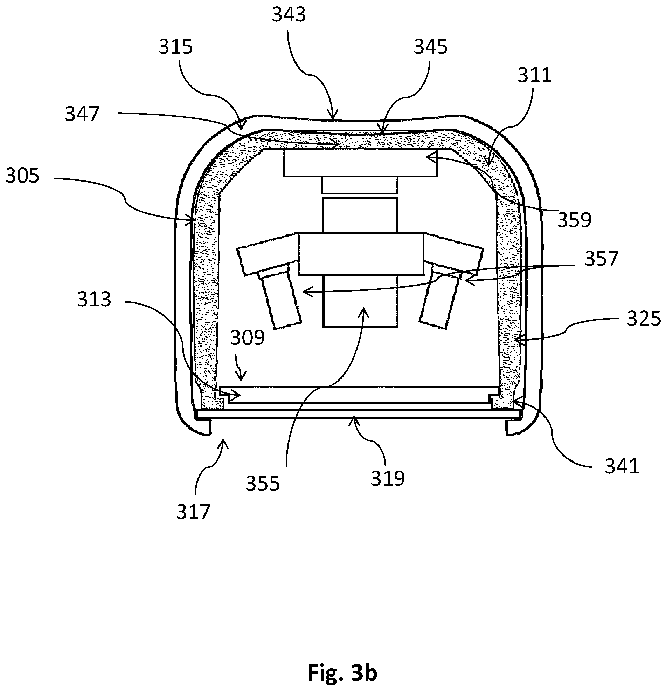

Fig. 3b illustrates a cross sectional view from a first perspective such as front view of the head and sleeve fully mounted on the housing according to an embodiment. This figure shows a heating element and positioning of illumination unit that is different from the one disclosed in Fig. 1, which illustrates a heating unit 123 and an illumination unit (not shown) comprised in the main body 103. However, for the purpose of understanding the perspective, it is to be understood that the front view is in the direction X (see Fig. 1), through the distal section of the head 305 of the intraoral scanner system 101 when the sleeve 315 is fully mounted, i.e. in position, onto the housing 107 with the primary aperture 309 and secondary apertures 317 in alignment with each other. The heating unit includes at least one component in the housing such as in the head 305, the at least one component being configured to generate heat in response to the at least one component performing a scanning related function during scanning of the patient. The at least one component may be an illumination unit 355 such as one or more LEDs that are configured to generate light, i.e. scanning related function, for illuminating an object to be scanned. The at least one component may be an image sensor 357 configured to generate the plurality of images based on the incoming (reflected) light received from the illuminated object, i.e. scanning related function. Placing the illumination unit and/ or sensor in the head allows projecting the light directly from the head onto the object and/ or to acquire data at the sensor directly from the reflected light. However, in response to receiving electrical power to generate the light, the light source also generates heat that is transferred, through the connection component, to the secondary optical component in the sleeve by way of thermal conduction during scanning. The heat generated by the at least one component during the scanning may be understood as a waste or residual heat that gets generated when the at least one component performs scanning related function during scanning. The at least one component may be connected to the connection component 325 using a contact component 359 that provides a direct interface (e.g. illumination unit 355) or indirect interface (e.g. image sensor 357) between the at least one component and connection component. Alternatively, the at least one component may be attached directly onto the connection component 325 with the contact component 359. A number of components in this embodiment are same and work on the same principle as those disclosed in the embodiment of Fig. 3a unless mentioned otherwise or are contradictory to utilizing waste or residual heat from the at least one component. For example, 311 represents a framework, 343 represents a pocket, 345 represents primary engagement elements, 347 represents secondary engagement elements, 325 represents the connection component but with a different geometry as illustrated, e.g. running along at least in part along an inner surficial periphery of the secondary engagement elements 347 of the head to establish conductive contact between the at least one component and the secondary optical component 319, 341 represents contact interface, 319 represents the secondary optical component and 313 represents the primary optical component. The weight of the connection component is kept low, but is generally higher 1.25 g, and usually below 5g. Surface roughness of the connection component 325 such as the profile roughness parameter (Ra) at interface(s) to conduct heat is usually kept as low as possible. There may be manufacturing limitation that affects how low profile roughness parameter (Ra) value may be achieved. The profile roughness parameter (Ra) may be in the range of 0.100 - 3.00 µm such as around 2.00 µm.Additionally of alternatively, Maximum Surface roughness (Rm) of the connection component at interface(s) to conduct heat is typically kept low and may be below 12 µm, such as below preferably below 10 µm, more preferably below 8 µm, even more preferably below 6 µm, most preferably below 4 µm. Although it may be preferred to keep both Maximum Surface roughness and the Average

Surface Average low, it is generally preferred to use the Maximum Surface roughness as a more relevant criterion.

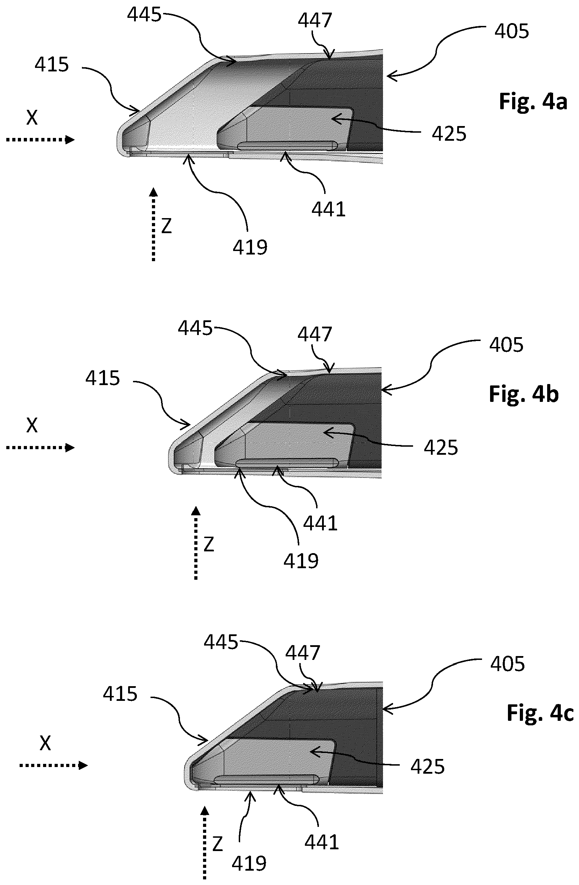

Fig. 4a illustrates, from a first view such as side view, a first stage during mounting of the sleeve onto the housing according to an embodiment. Fig. 4b illustrates, from the first view such as side view, a second stage during mounting of the sleeve onto the housing according to an embodiment. Fig. 4c illustrates, from the first view such as side view, a third stage during mounting of the sleeve onto the housing according to an embodiment. The side view is a view in the direction Y (see Fig. 1). The first stage, second stage and third stage refer to different stages of the intraoral scanner system 101 during the process of mounting the sleeve 415 onto the housing 107. Fig. 4a shows a situation just before the secondary optical component 419 is brought into contact with the connection component 425 of the defogging system 227. Fig 4b shows a stage where the sleeve 415 is partially mounted on the head 405. The connector interface 441 of the connection component 425 has received, in part, the secondary optical component 419 and is guiding the secondary optical component into position. Fig 4c shows the fully mounted stage where the primary engagement element 445 and secondary engagement elements 447 are in alignment and the secondary optical element is correctly positioned and in physical contact with the connection component 425, thereby placing the secondary optical component such that thermal conduction via connection component is possible.

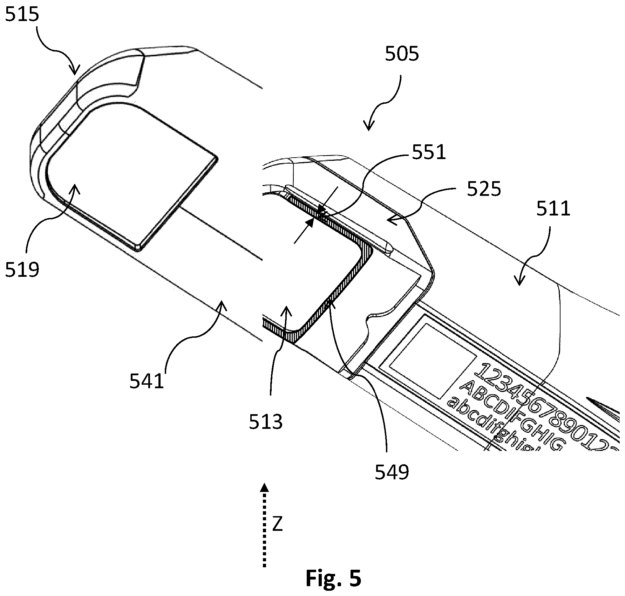

Fig. 5 illustrates, from the second view such as bottom view (in the direction of Z), the first stage (see Fig. 4a) during mounting of the sleeve onto the housing according to an embodiment. When the sleeve 515 is fully mounted, the connection component 525 is configured to create a contact area 549, outside the propagation area through which the scanning signal passes, with the secondary optical component 519, thus establishing physical contact for thermal conduction of the generated heat. The heat is then distributed through the entire area 549 along the peripheral section of the secondary optical component 519 such that heat can flow toward the center of the secondary optical component 519 to prevent fogging during scanning. The heating time of the secondary optical component 519 is dependent on the contact area, which is a function of the width of the runners 551 in the contact interface 541. It is apparent that for same length of the runners 551, a greater width would provide a greater contact area, thereby providing a more effective heating. The width of the runners 551 may be in the range of 0.2 to 2.0 mm, preferably around 1mm such as 0.8 mm. As the efficiency of the thermal conduction may depend upon surface roughness, the choice of width may thus depend upon the Ra value. One other consideration is mechanical integrity, i.e. that choice of width and/ or runner design ensure that the secondary optical component is prevented from getting chipped during engagement or disengagement. Fast and efficient heating of the surfaces of the secondary optical element may thus be achieved by having an intimate connection between the defogging unit 121, the connection component 525 and the secondary optical component 519.

Several aspects, discussed below in relation to Fig. 1 or other relevant figure, may be considered to optimize the heat transfer. These aspects are not limited to this embodiment but may also be used in combination with other embodiments:
First, the primary function of the connection component is to thermally conduct the generated heat from the heating unit 123 to the secondary optical component 119. As disclosed earlier, the connection component comprises a material having a high thermal conductivity. Accordingly, the connection component may allow transfer of, from the heating unit 123, the generated heat, which may get distributed throughout the entire connection component 125. Accordingly, physical characteristics of the connection component 125 may also determine effectiveness of heating of the secondary optical component. The physical characteristic may include one or more of shape, thickness, volume, mass, etc. It is preferred that the connection component 125 is designed such that the connection component is prevented from extending in regions that do not contribute to transferring heat to the secondary optical component 119. In other words, the connection component is designed such that the connection component extends primarily, such as only, towards the secondary optical component. Such extension of the connection component may provide the shortest path from the heating unit 123 to the secondary optical component 119. By altering the physical characteristic, e.g. reducing the volume of the connection components at specific regions, transfer of the generated heat may be controller and heating the secondary optical element may be achieved faster. As an example, a connection component that extends primarily, such as only, towards the secondary optical component as shown as 125 in comparison to a connection component having a shape that also goes around region 131 (Fig. 1) of the head, i.e. reducing the connection component coverage in mass by approximately 70% or more, may reduce the defogging time by at least 35%.

Additionally, the connection component 125 may define the primary aperture 109, thus the connection component may further be configured to support the primary optical component 113. In view of multi-functional role of the connection component, different portions of the connection component may include different physical characteristics. The portion of the connection component providing the support may have a different physical characteristic in comparison to that of the portion of the connection component that is primarily functioning to thermally conduct heat to the secondary optical component. For example, the portion supporting the primary optical component may be thicker than the portion designed primarily to transfer heat to the secondary optical component.

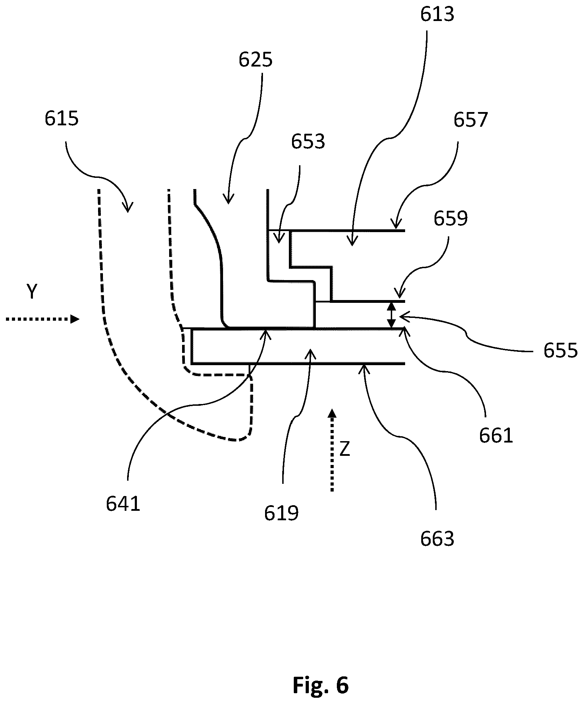

Second, the transfer of the generated heat to the primary optical component 113 may also be considered. As described previously, the connection component 125 may include a mounting frame, defining the primary aperture, for arranging the primary optical component 113. Thus, the connection component may provide mechanical support to mount the primary optical component at the primary aperture, which is defined by the connection component. This is illustrated in Fig. 6, which shows a zoomed cross-sectional view of the fully mounted sleeve 115 on the head 105, as seen from front in the direction X, according to an embodiment. This illustration corresponds to Figs. 3a and 3b. Fig. 6 illustrates a detailed interface between the connection component 625, the primary optical component 613 and the secondary optical component 619 that is arranged in the sleeve 615. In order to inhibit the generated heat from unnecessarily transferring to the primary optical component 613, the thermal connection between the connection component 625 and the primary optical component 613 may be restricted. This is done because heating the primary optical component would increase the time or power it takes to reach the preferred temperature on the secondary optical component 625 and to achieve defogging. The primary optical component 613 may be mounted at the connection component 625 using a thermal isolating bonding adhesive 653 such as silicone, epoxy or UV curing glues which will form a heat isolating barrier thereby preventing the generated heat from being transferring from the heating unit 223 to the primary optical component 613 while simultaneously fixating the primary optical component 613 securely in place in the frame of the connection component 625.

Third, it is also useful to reduce, preferably minimize, the heat radiated between the secondary optical component 619 and the primary optical component 613. When the sleeve 615 is fully mounted onto the head, the secondary optical component 619 is is fully engaged (i.e. in desired physical contact) with the connection component 625 at the contact interface 641. Apart from ensuring good physical contact for thermal conduction of the generated heat to the secondary optical component, this engagement/ arrangement also ensures that the that the primary second surface 659 of the primary optical component 613 is separated from the secondary first surface 661 of the secondary optical component 619 by a gap 655. 657 represents primary first surface of the primary optical component 613 and 663 represents secondary second surface of the secondary optical component 619. The distance or gap width 655 between the primary optical component 613 and secondary optical component 619 secures that direct contact between these components is avoided. It is preferred that the distance 655 between the two optical components (613, 619) is as large as possible to inhibit heat transfer between these components such as from the secondary first surface 661 of the secondary optical component 619 by convection or radiation to the primary second surface 659 of the primary optical component 613. It may be appreciated that at least a part of the sleeve 615 and at least a part of the head unit 105 (e.g. the distal end section) is intended to be inserted into the oral cavity, offering limited space, to acquire 3D information. Therefore, there is a need to have as smallest probe head size comprising the distal end section as possible. The small size offers the advantage of easy maneuverability of the head (105) within the intraoral cavity and obtaining 3D information from many different places and angles without discomfort to the patient. In view of the conflicting requirements, it is determined that a gap width 655 between the optical components (613, 619) in the range [0.1 to 1 mm, preferably less than 0.5 mm such as 0.2 mm produce an effective defogging of the secondary optical component.

Fourth, heat transfer at the secondary optical component 619 needs to be high. In other words, the generated heat received by the secondary optical component 619 at the contact interface 641 needs to be transferred as quickly as possible across the secondary optical component (619). Thus, the received generated heat needs to be transferred from the edges of the secondary optical component 619 inwards towards the center to achieve a temperature distribution above the dew point of the scanning environment. This may be achieved by designing the secondary optical component 619 by selecting an optically transparent material with an intrinsically high thermal conductivity. Additionally, heat transfer in the primary optical component 613 may be as low as possible to further support the suppression of heat flowing directly or indirectly from the heating unit to the primary optical component 613. Thus, the material for the primary optical component 613 may be selected from optically transparent materials with an intrinsically low thermal conductivity.

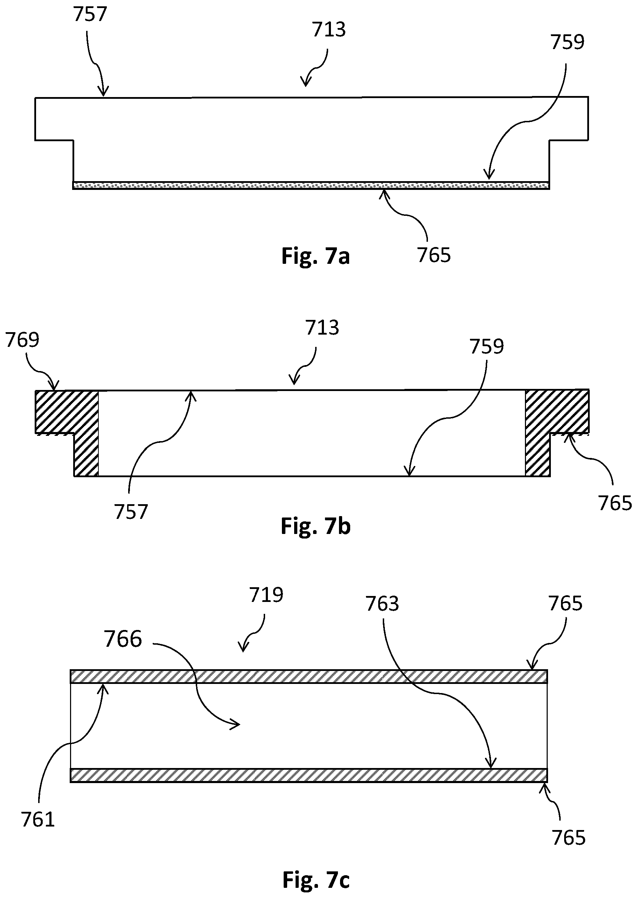

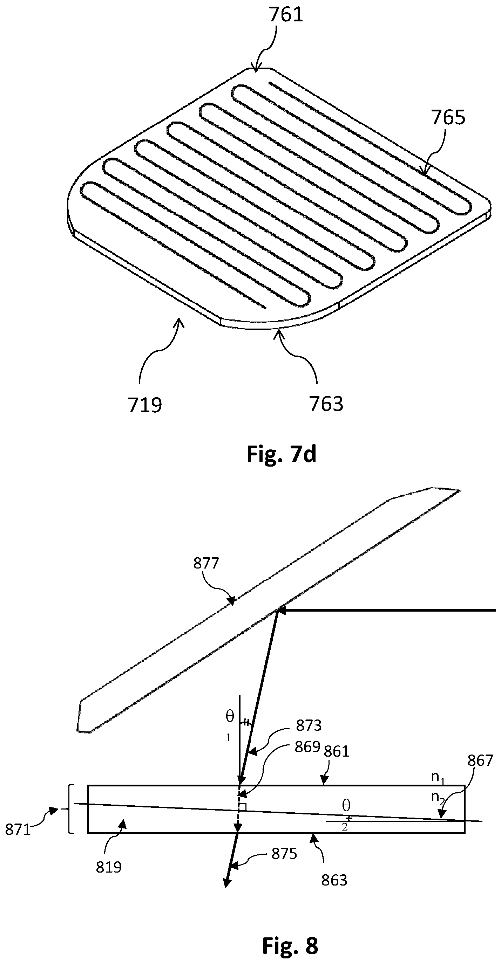

In an embodiment, at least one of the primary optical components or the secondary optical component may be designed in a layered configuration. The layered configuration may be designed to provide beneficial characteristics in relation to thermal conductivity and heat transfer, as described above. This is illustrated in Fig 7, which illustrate optical components according to different embodiments.

Referring to Fig. 7a, the primary optical component 713 may include a substrate having a deposit of poor thermal conductivity or insulating properties. At least one of the primary first surface 757 or primary second surface 759 of the primary optical component 713 may act as a substate with the deposit disposed over the at least one of the primary first surface 757 or primary second surface 759. Thus, use of such deposit prevents the generated heat from heating up the primary optical component 713 and/ or inhibit radiated heat from the secondary optical component 719 across the gap width to heat up the primary optical component 713. In an example, the primary optical component may be made of BK7 optical grade glass with a nano transparent heat insulating coating deposit as the deposit 765 on the primary second surface 769 of the primary optical component 713. The deposit 765 may by a transparent heat insulating coating composed of antimony doped tin oxides (ATO) nanoparticles or any other suitable ceramic or metallic coatings. The deposit 765 may alternatively be composed of an ultra-thin polyester-based film. Alternatively, the substrate may include a material having a low thermal conductivity, such that heat is dispersed slower in the deposit compared to the bulk of the primary optical component 713. Such a material may be bonded to the bulk material by UV glue and be an optical transparent polymer like and amorphous thermoplastic such as Polymethylmethacrylate (PMMA) or Polycarbonate (PC).

Referring to Fig. 7b, the substrate includes edges of the primary optical component 713 and the deposit 765 is located along the edges of the primary optical component 713. In this case, the deposit may be composed of a material having a low thermal conductivity, such that heat generated by the heating unit is inhibited from flowing, across the mechanical interface defined the deposit, to the primary optical component 713.

Fig 7c illustrates a multi-layered secondary optical component according to the embodiment. The secondary optical component 719 includes several layers contributing to its heat transfer ability across its surface. The secondary optical component 719 may include a substrate having a deposit 765 of high thermal conductivity. At least one of the secondary first surface 761 or secondary second surface 763 of the secondary optical component 719 may act as a substate with the deposit 765 disposed over the at least one of the secondary first surface 761 or secondary second surface 763. Thus, use of such deposit 765 facilitates the generated heat of being trnasferred throughout the secondary optical component 719 such that at least the secondary second surface 763 may reach the desired temperature as fast and efficiently as possible to achieve defogging. In an example, the secondary optical component 719 includes a layered structure of optical grade crown glass 766, like borosilicate glass (BK7) sandwiched in between deposit 765 composed of thin layers of corundum like sapphire glass. The thin layers of sapphire glass may have in intrinsically higher thermal conductivity then the crown glass core 766, thus facilitating the transfer of heat along the surface of the secondary optical component 719.

Referring to Fig. 7d, the secondary optical component 719 may include, such as embedded or over the surfaces, wires as deposit 765 for the purpose of defogging. The wires may be provided at least one of the secondary first surface 761 or seondary seond surface 763 of the secondary optical component 719. The wires may be adhered to at least one surface of the secondary optical component. Additionally or alternatively, the wires may be embedded within the secondary optical component, i.e. sandwiched between the secondary first surface and secondary second surface. The wires may be separated by a distance along the length and/ or width of the secondary optical component 719, such as equidistant from one another. Such wires 765 may be composed of a material having high thermal conductivity, such as copper, gold, Nickel chromium or alike. One or more wires may be configured to be in physical contact with the connection component when the sleeve is in mounted over the head, i.e. in fully received position with the secondary optical component being in alignment with the primary aperture. When the wires comes in contact with connection component 125, the generated heat from the heating unit 123 is configured to be conducted through the wires to efficienlty heat up the secondary optical component in a fast manner. The transport of thermal energy in a material is usually governed by one or more of material properties, heat flux and temperature difference. In an example using a secondary optical component of size 25 X 18 mm and 30 copper wire strips along the 25 mm length and width of 20 µm and positioned at a distance of 600 µm between adj acent wires, a fill factor (f) of approximately 3% is achieved. In another example using a secondary optical component of size 25 X 18 mm and 30 gold wire strips along the 18 mm length and width of 20 µm and positioned at a distance of 800 µm between adjacent wires, a fill factor (f) of approximately 2.5% is achieved. Fill factor may be defined as the percentage of the secondary optical component area (e.g. surface area), covered by the wires, that is otherwise exposed to the scanning signal. Inclusion of the wires produce opaqueness, which may start interfering with the scanning signal if the fill factor exceeds a certain level. The skilled person would realize that a higher fill factor may make the heating of the secondary optical copmonent more efficient (i.e. reduced defogging time) but at the expense of increaing area covered with wire. However, it is determied that a fill factor of below 12% such as between 2 to 12% produces negligible impact on the scanning signal. Thus, a fill factor in the disclosed range ensure that the scanning signal does not get altered substanitally to negatively affect the data acquired during scanning. Furthermore, the sharpness of the acquired two-dimensional image may essentially be unaffected by presence of the wires as well. Such a deposit configuration using wires at the secondary optical component introduces the capability of transferring heat across the secondary optical component without the requirement of the secondary optical component necessarily having a high thermal conductivity. This allows a wider range of material or conifugration choices for the secondary optical component. For example, the secondary optical component 719 may be made out of non-birefringent Polymethylmethacrylat (PMMA) or polycarbonate (PC) with a wire deposit 765 on surface of the secondary optical component.

A sleeve, deteachable from the housing, may be used as a multi-use sleeve or a single use sleeve. The secondary optical component 719 may be detachably mounted in a multi-use sleeve or in a single use sleeve. For multi-use sleeve, at least the sleeve 115 need to to withstand reprocessing (typically autoclaving, wiping with alcohol, washing with soft brush and soap etc.) in between usage on different patients. The secondary optical component 119 may be replaceable between usages or be confirgured to withstand the reprocessing as well.

The secondary optical component 119 may additionally be able to withstand disinfection if the sleeve 115 comprising a permanently attached secondary optical element 119 is intended to be used multiple times. Disinfection or sterilization is a step in the reprocessing of reusable dental instruments that have become contaminated, and is typically performed in a steam autoclave, which is typically performed at 134 - 137°C, 2.1 - 2.25 bar gauge pressure for at least a 3 minutes. Typically, a sleeve 115 may be used for up to 170 repeated cycles. The autoclave process may cause the light transmission properties to decay as the surfaces of the secondary optical component (119) may deteriorate over time. Multiple autoclave cycles of the sleeve 115 may also leave residues on the secondary first surface 661 and/or secondary second surfaces 663 which may cause irreversible changes as it may be very hard to clean (e.g., metal oxides, calcium carbonate or organic material). Therefore, the secondary first surface and/ or secondary second surface may be coated with hydrophobic coating such as highly hydrophobic coating like Perfluorodecyltrichlorosilane (FDTS). The coating causes droplets of condensed steam to be repelled from the surfaces (661,663), rather than sticking and drying, causing deposits on the surfaces (661,663). It may be beneficial to place the sleeve 115 in the autoclave, such that the secondary optical component 119 is resting in a vertical position relative to gravity in order to allow droplets to roll off before evaporating and leaning deposits. Should deposits form on the surfaces (661,663), the FDTS coating makes the deposits much easier to be removed, thereby avoiding irreversible deterioration of secondary optical element 119.

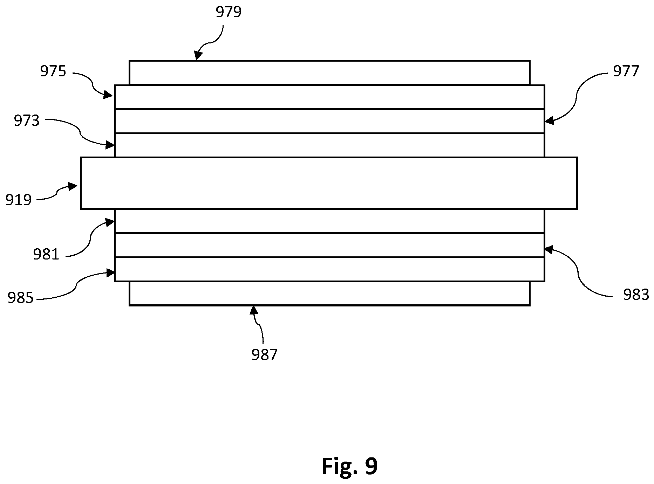

At least one of the primary optical component 113 or secondary optical component 119 may be equipped with anti-reflective (AR) coating. In an example, two-layer coating such as layers of Ta₂O₅ and SiO₂ on one or more surfaces of at least one of the primary optical component or secondary component component may be used such as on the surfaces (659, 661, 663), which are required to withstand the cleaning process, and in some cases even autoclaving. The AR coating may have a reflectance, averaging below 1% in the visible spectrum. As the outer layer may be SiO₂, additional coatings such as Perfluorodecyltrichlorosilane (FDTS) readily forms strong covalent bonds to the surface. An intermediate bonding layer such as aluminum oxide, may also be used between the outer layer and additional coating. The intermediate bonding layer may be configured to promote adhesion and may be deposited using layer deposition techniques such as atomic layer deposition. The interior surface of the framework 115 may additionally be equipped with a 3-layer AR coating, such as layers of Ta₂O₅, SiO₂ and MgF₂, with broad band low reflection and average reflection for visible light may be below 0.5% to avoid stray light inside the scanner. This is shown by way of Fig. 9 that illustrates a multilayered secondary component according to an embodiment. The secondary optical component 919 includes two layers of coating of Ta₂O₅ (973, 981) and SiO₂ (977, 983) on both surfaces of the secondary optical component. Additional hydrophobic coating of Perfluorodecyltrichlorosilane (FDTS) (979, 987) are also provided over the coated layers on either surfaces of the secondary optical component with an intermediate bonding layer of Al₂O₃ (975, 985) being sandwiched between the hydrophobic coating (979, 987) and coated layers (973-975, 981-985).

In an embodiment, the optical system of the scanner is configured to operate using polarized light signals. Thus, the primary optical component 113 and secondary optical component 119 may be configured to substantially maintain the polarization state of the light transmitted through these components (113, 119). Additionally, the secondary optical component 119 is configured to be heated quickly to allow effective defogging of the secondary optical component. To achieve the intended effective defogging, the secondary optical component may include a crystalline structure that typically offers significantly faster heating when compared to an amorphous glass material. For example, Corundom like Sapphire may be chosen as a suitable crystalline structure material for the secondary optical component because of its high thermal conductivity. Although crystalline structure like Sapphire may offer the advantage for heating, but it may intrinsically be birefringent, and may thus deteriorates the polarization state of transmitted polarized light. To mitigate this, now referring to Fig. 8, the secondary optical element 819 like sapphire is configured such that the light passing through the secondary optical component is perpendicular to crystal plane (867, c-plane (0 degree) - 0001 orientation) of the crystalline structure. The light passing through the crystalline structure is defined by an optical axis 869, i.e. light path within the crystalline structure. The mirror 877 is configured to allow directing the scanning signal out of the scanner and/ or towards the sensor. Alternatively, a glass prism may replace the mirror to allow directing the scanning signal.

In one embodiment, for a predefined physical arrangement of scanner components with sleeve in the mounted state onto the head and positioning of the secondary optical component with respect to optical axis (873, 875) known, configuring the crystalline structure includes designing the crystal lattice of the crystalline structure such that the light passing through the secondary optical component is perpendicular to the crystal plane. For example, if the secondary optical component 819 is positioned at an angle θ₁ relative to the optical axis 873 of the scanner at the secondary first surface 861 (863 represents the secondary second surface), then the crystalline structure is cut at an angle to the c-plane such that the c-plane will be perpendicular to the optical axis 869.

In another embodiment, for a given crystalline structure having a predefined crystal plane, configuring the crystalline structure includes designing physical arrangement of scanner components for sleeve in the mounted state onto the head and positioning of the secondary optical component with respect to optical axis (873, 875) such that the light passing through the secondary optical component is perpendicular to the predefined crystal plane when sleeve is mounted onto the head. For example, the secondary first surface and secondary second surface of the secondary optical component 819 is configured to be at an angle θ₂ with respect to the c-plane, such that the angle θ₂ corresponds to the internal light propagation angle inside the crystal being perpendicular to the C-plane 867 of the crystal. The angle θ₂ is a function of the angle of incident light and the ratio between the refractive index (n1, n2) of the materials on each side of the interface (e.g. the secondary first surface 861). The angle θ₂ may be applicable in both disclosed embodiments, i.e. to design the crystalline structure and/ or arranging a given secondary optical component in the scanner. In one scenario, the secondary optical component 819 is interfaced at secondary first surface 861 by air and angled at θ₁ which may be in the range of 12° to 16° such as 14° and angle θ₂ may be in the range of 6° to 10° such as around 7.5°. In another scenario, the secondary optical component may be interfaced at the secondary first surface 861 with another material such as a glass prism or alike. The value of at θ₁ and angle θ₂ may be adjusted to achieve the light passing through the secondary optical component to be perpendicular to the predefined crystal plane.

Although the disclosure is made in relation to probe light exiting the scanner and illuminating the dental object; the disclosed principle in relation to configuring the crystalline structure may equally be applicable for the reflected light received in response to illumination of the dental object. With the disclosed configuration, the birefringence may be minimized such that using the crystalline structure for the secondary optical component does not substantially alter the transmitted light.

Additionally, the negative impact of using crystalline structure like sapphire as the secondary optical component 819 on the polarization state of the probe light may additionally be correlated to thickness 871 of the crystalline structure 819. Preferably the crystalline structure needs to be as thin as possible while still being sturdy during use such as scratch resistance and not break easily upon accidental contact with teeth, and for handling during fabrication and assembly. As the percentage light transmitting across the secondary optical component with desired polarization is a function of the thickness of the secondary optical component, it is preferred that the secondary optical component is as thin as possible. It is determined that the secondary optical component 819 may have a thickness of in the range of 0.1 mm to 1mm, such as 0.5 mm. Having a thickness in this range does not change the polarization substantially to impact the scan data quality, while still being sturdy for the purpose of intraoral scanning. It is also preferred to having the secondary optical component made of crystalline glass structure such as a sapphire. In the scenario where the secondary optical component breaks, another advantage is that sapphire will typically form a clean break, as it is crystalline, resulting in fewer, larger pieces of debris. An amorphous glass will shatter an many small sharp pieces as it breaks.

### List of Items

1. An intraoral scanning system comprising:
   a housing comprising
   optical components,
   a head, comprising a primary aperture, configured to be inserted into an oral cavity of a patient, and
   a defogging unit comprising a heating unit;
   a sleeve configured to cover at least a part of the head when the sleeve is coupled with the housing, the sleeve comprising a secondary optical component arranged at a secondary aperture that is configured to be positioned in alignment with the primary aperture, wherein
   the heating unit is configured to generate heat in response to application of electrical power to the heating unit, and
   the secondary optical component is configured to be arranged such that the generated heat is transferred from the heating unit to the secondary optical component through thermal conduction between the heating unit and secondary optical component.
2. The system according to item 1, wherein the housing comprises a connection component that is configured to establish a physical connection between the heating unit and the secondary optical component to permit transfer of the generated heat by thermal conduction from the heating unit to the secondary optical component.
3. The system according to any one or more of the preceding items, wherein the heating unit is arranged on the connection component.
4. The system according to any one or more of the preceding items, wherein the heating unit is physically connected to the connection component using a thermal conductive material.
5. The system according to any one or more of the preceding items, wherein the sleeve is removably attached with the housing such that coupling the sleeve with the housing arranges the secondary optical component in physical connection with the heating unit via the connection component for permitting thermal conduction of the generated heat from the heating unit to the secondary optical component.
6. The system according to any one or more of the preceding items, wherein the secondary optical component is made up of a material having a thermal conductivity higher than the thermal conductivity of the primary optical component.
7. The system according to any one or more of the preceding items, wherein the secondary optical component comprises a substrate comprising a deposit of high thermal conductivity disposed on at least one of a secondary first surface or secondary second surface of the secondary optical component.
8. The system according to any one or more of the preceding items, wherein the secondary optical component comprises a multi-layered structure where at least two layers of the multi-layered structure have different thermal conductivity and/ or optical properties.
9. The system according to any one or more of the preceding items, further comprising a primary optical component arranged at the primary aperture, the primary optical component being inhibited from receiving the generated heat from the heating unit to the primary optical component.
10. The system according to any one or more of the preceding items, further comprising an insulating material arranged between the primary optical component and the heating unit to inhibit transfer of the generated heat from the heating unit to the primary optical component.
11. The system according to any one or more of the preceding items, wherein the primary optical component is made up of a material having a thermal conductivity lower than the thermal conductivity of secondary optical component.
12. The system according to any one or more of the preceding items, wherein the primary optical component comprises a substrate comprising a deposit of poor thermal conductivity disposed on at least one of a primary first surface or a primary second surface of the primary optical component.
13. The system according to any one or more of the preceding items, wherein the primary optical component comprises a multi-layered structure where at least two layers of the multi-layered structure have different thermal conductivity and/ or optical properties.
14. The system according to any of the preceding items, wherein the thermal conductivity of the primary optical component material is lower than the thermal conductivity of the i) connection component, or ii) connection component and secondary optical component.
15. The system according to any of the preceding items, wherein the thermal conductivity of the insulating material arranged between the primary optical component and the heating unit is lower than the thermal conductivity of the i) connection component, or ii) connection component and secondary optical component.
16. The system according to any of the preceding items, wherein the thermal conductivity of the deposit applied on the primary connection component is lower than the thermal conductivity of the i) connection component, or ii) connection component and secondary optical component.
17. The system according to any of the preceding items, wherein thermal conductivity of materials defining a thermal path from the heating unit to the secondary optical component is higher than the thermal conductivity of materials defining a thermal path from the heating unit to the primary optical component.
18. The system according to any one or more of the preceding items, wherein
   when the sleeve is coupled with the housing, a relative position of the primary optical component and secondary optical component defines a gap therebetween, and
   the gap comprises a gap-width that allows thermal insulation between the primary optical component and secondary optical component.
19. The system according to any one or more of the preceding items, wherein the gap width is at least partially filled with a thermal insulating material.
20. The system according to any one or more of the preceding items, wherein
   the head comprises a primary engagement element and the sleeve comprises a secondary engagement element; and
   the primary engagement element and secondary engagement element are configured to physically interact during coupling of the sleeve with the housing to bring the connection component and secondary optical component/ a secondary frame comprising the secondary optical component in a physical connection with each other.
21. The system according to any one or more of the preceding items, wherein
   the head comprises a primary engagement element and the sleeve comprises a secondary engagement element; and
   the primary engagement element and secondary engagement element are configured to physically interact such that the connection component and secondary optical component/ the secondary frame comprising the secondary optical component stay in a physical connection with each other when the sleeve is coupled with the housing.
22. The system according to any one or more of the preceding items, wherein the physical connection between the connection component and secondary optical component/ the secondary frame comprising the secondary optical component is defined by a contact surface area at an interface between the connection component and secondary optical component/ the secondary frame.
23. The system according to any one or more of the preceding items, wherein a portion of the connection component interfacing the secondary optical component/ the secondary frame comprising the secondary optical component is dimensioned such that when the sleeve is coupled with the housing, the connection component is restricted from interfering with the signal receiving section of the secondary optical component.
24. The system according to any one or more of the preceding items, wherein when the sleeve is coupled with the housing, a portion of the connection component interfacing the secondary optical component/ the secondary frame comprising the secondary optical component is arranged in relation to the secondary optical component/ the secondary frame to avoid the connection component interfering with a signal receiving section of the secondary optical component.
25. The system according to any one or more of the preceding items, wherein
   one of the primary engagement element or secondary engagement element comprises a protrusion and another of the primary engagement element or secondary engagement element comprises a surface that is configured to physically interact with the protrusion; and
   the protrusion and the surface are configured to physically interact to bias the secondary optical element/ secondary frame comprising the secondary optical element and connection component towards each other.
26. The system according to any one or more preceding items, wherein
   one of the primary engagement element or secondary engagement element comprises a guide and another of the primary engagement element or secondary engagement element comprises a guide channel that is configured to receive the guide; and
   movement of the guide along the guide channel is configured to bias the secondary optical element/ secondary frame comprising the secondary optical element and connection component towards each other.
27. The system according to any one or more preceding items, wherein the heating unit comprises an embedded heater that is configured to solely generate heat prior to and/ or during scanning of the patient's teeth.
28. The system according to any one or more preceding items, wherein heating unit includes at least one component in the housing, the at least one component being configured to generate heat in response to the at least one component performing a scanning related function during scanning of the patient.
29. The system according to any one or more preceding items, wherein the scanning related function is different from solely to generate heat.
30. The system according to any one or more preceding items, wherein the at least one component is configured to generate heat comprising residual or waste heat.
31. The system according to any one or more preceding items, wherein average surface roughness of the connection component at interface(s) to conduct heat is in the range of 0.100 - 3.00 µm.
32. The system according to any one or more preceding items, wherein maximum surface roughness of the connection component at interface(s) to conduct heat is below 12 µm.
33. The system according to any one or more preceding items, wherein the head of the housing comprises a temperature sensor.
34. The system according to any one or more preceding items, wherein the temperature sensor is arranged proximal to the primary aperture.
35. The system according to any one or more preceding items, wherein the temperature sensor is configured to measure temperature in the head and provide sensor measurements as signal to a control unit.
36. The system according to any one or more preceding items, wherein the control unit, based on the received sensor measurement signal, is configured to provide feedback control signal.
37. The system according to any one or more preceding items, wherein the control unit provides the feedback control signal at least when the sensor measurement signal is beyond a threshold temperature value.
38. The system according to any one or more preceding items, wherein the threshold temperature value may be predefined based on a permissible temperature in the oral cavity.
39. The system according to any one or more preceding items, wherein the feedback control signal is configured to put the scanner in a throttling mode.
40. The system according to any one or more preceding items, wherein, the feedback control signal is configured to control the temperature setting of the embedded heating unit such that the sensor measurement at the temperature sensor is at or within the threshold temperature value.
41. The system according to any one or more preceding items, wherein the secondary optical component comprises layers of coating of Ta₂O₅ and SiO₂ on at least one surface of the secondary optical component.
42. The system according to any one or more preceding items, wherein the secondary optical component comprises hydrophobic coating of Perfluorodecyltrichlorosilane (FDTS) over the layers of coating.
43. The system according to any one or more preceding items, further comprising an intermediate bonding layer of Al₂O₃ sandwiched between the hydrophobic coating and coated layers.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the present invention. It may be understood that the comparative statements mentioning higher/ lower including variations thereof, or disclosed range/ values for comparative parameters or properties such as thermal conductivity and/ or heat capacity are made in relation to substantially same environment conditions, for example room temperature or intraoral environment.

Benefits, other advantages, and solutions to problems have been described herein with regard to specific embodiments. However, the benefits, advantages, solutions to problems, and any component(s)/ unit(s) that may cause any benefit, advantage, or solution to occur or become more pronounced are not to be construed as critical, required, or essential features or components/ elements of any or all the claims or the invention. The scope of the invention is accordingly to be limited by nothing other than the appended claims, in which reference to an component/ unit/ element in the singular is not intended to mean "one and only one" unless explicitly so stated, but rather "one or more." A claim may refer to any of the preceding claims, and "any" is understood to mean "any one or more" of the preceding claims.

As used, the singular forms "a," "an," and "the" are intended to include the plural forms as well (i.e. to have the meaning "at least one"), unless expressly stated otherwise to be limited to "only" one/ single. It should be emphasized that the term "comprises/ comprising/ including/ having" when used in this specification is taken to specify the presence of stated features, integers, operations, steps or components but does not preclude the presence or addition of one or more other features, integers, steps, components or groups thereof. As used herein, the term "and/or" includes any and all combinations of one or more of the associated listed items. The steps of any disclosed method are not limited to the exact order stated herein, unless expressly stated otherwise.

In claims enumerating several means, several of these means can be embodied by one and the same item of hardware. The mere fact that certain measures are recited in mutually different dependent claims or described in different embodiments does not indicate that a combination of these measures cannot be used to advantage.

## Claims

1. An intraoral scanning system (101) comprising:
• a housing (107),
• optical components (113, 119) that include a primary optical component (113) and a secondary optical component (119), wherein the primary optical component (113) is arranged inside the housing (107),
• a head (105), comprising a primary aperture (109), configured to be inserted into an oral cavity of a patient, and
• a defogging unit (121) comprising a heating unit (123);
• a sleeve (115) configured to cover at least a part of the head when the sleeve (115) is coupled with the housing (107), the sleeve (115) comprising the secondary optical component (119) arranged at a secondary aperture (117) that is configured to be positioned in alignment with the primary aperture (109), and
wherein the heating unit (123) is configured to generate heat in response to application of electrical power to the heating unit (123), and the secondary optical component (119) is configured to be arranged such that the generated heat is transferred from the heating unit (123) to the secondary optical component (119) through thermal conduction between the heating unit (123) and secondary optical component (119), wherein the housing (107) comprises a connection component that is configured to establish a physical connection between the heating unit (123) and the secondary optical component (119) to permit transfer of the generated heat by thermal conduction from the heating unit (123) to the secondary optical component (119).

2. The system (101) according to claim 1, wherein the secondary optical component (119) comprises a substrate comprising a deposit (765) of high thermal conductivity disposed on at least one of a secondary first surface (761) or secondary second surface (763) of the secondary optical component (119).

3. The system (101) according to any one or more of the preceding claims, further comprising a primary optical component arranged at the primary aperture, wherein the primary optical component (113) being inhibited from receiving the generated heat from the heating unit (123) to the primary optical component (113).

4. The system (101) according to any one or more of the preceding claims, further comprising an insulating material arranged between the primary optical component (113) and the heating unit (123) to inhibit transfer of the generated heat from the heating unit (123) to the primary optical component (113).

5. The system (101) according to any one or more of the preceding claims, wherein the primary optical component (113) comprises a substrate comprising a deposit (765) of poor thermal conductivity disposed on at least one of a primary first surface (657, 757) or a primary second surface (659, 759) of the primary optical component (113).

6. The system (101) according to claim 1, wherein the heating unit (123) is arranged on the connection component (125).

7. The system (101) according to any one or more of the preceding claims, wherein the thermal conductivity of the primary optical component material is lower than the thermal conductivity of the i) connection component (125), or ii) connection component (125) and secondary optical component (119).

8. The system (101) according to claim 4 and any of claims 5-7, wherein the thermal conductivity of the insulating material arranged between the primary optical component (113) and the heating unit (123) is lower than the thermal conductivity of the i) connection component (125), or ii) connection component (125) and secondary optical component (119).

9. The system (101) according to any of the preceding claims 5-8, wherein the thermal conductivity of a deposit (765) applied on the primary connection component (113) is lower than the thermal conductivity of the i) connection component (125), or ii) connection component (125) and secondary optical component (119).

10. The system (101) according to any of the preceding claims, wherein thermal conductivity of materials defining a thermal path from the heating unit (123) to the secondary optical component (119) is higher than the thermal conductivity of materials defining a thermal path from the heating unit (123) to the primary optical component (113).

11. The system (101) according to any one or more preceding claims, wherein average surface roughness of the connection component (125) at interface(s) to conduct heat is in the range of 0.100 - 3.00 µm.

12. The system (101) according to any one or more preceding claims 5-9, wherein maximum surface roughness of the connection component (125) at interface(s) to conduct heat is below 12 µm.

13. The system (101) according to any one or more preceding claims 5-9 or 11, wherein the housing (107) comprises a connection component (125) that is configured to establish a physical connection between the heating unit (123) and the secondary optical component (119) to permit transfer of the generated heat by thermal conduction from the heating unit (123) to the secondary optical component (119).

14. The system (101) according to any one or more preceding claims, wherein the optical components include one or more of light source, prism, mirror, lens system, beamsplitter, and transparent glass.

15. The system (101) according to any one or more preceding claims, wherein the heating unit (123) comprises an embedded heater that is configured to solely generate heat prior to and/ or during scanning of the patient's teeth.
